# EUROPEAN PATENT APPLICATION

(11) **EP 3 575 401 A1**
(43) Date of publication of application: **04.12.2019**
(21) Application number: 18174938.3
(22) Date of filing: 29.05.2018
(51) Int. Cl.: C12N 15/113, A61K 31/7088, A61K 48/00

(54) **METHODS OF TREATING OR PREVENTING AN AGING-RELATED DISEASE**

(71) Applicant: ECOLE POLYTECHNIQUE FEDERALE DE LAUSANNE (EPFL), 1015 Lausanne (CH)
(72) Inventor: D'AMICO, Davide, 1020 Renens (CH); AUWERX, Johan, 1164 Buchillon (CH)
(74) Representative: KATZAROV S.A.

(57) **Abstract**

The invention relates generally to methods, agents and pharmaceutical compositions for treating or preventing an aging-related disease by reducing the levels and/or activity of the RNA-binding protein PUM2.

SEQUENCE LISTING

<110>  Ecole Polytechnique Fédérale de Lausanne (E.P.F.L.)

<120>  Methods of treating or preventing an aging-related disease

<130>  51375/EP

<160>  29

<170>  PatentIn version 3.5

<210>  1
<211>  20
<212>  DNA
<213>  Homo sapiens

<400>  1
ttgactttat tcatccattt                                                    20

<210>  2
<211>  21
<212>  DNA
<213>  Homo sapiens

<400>  2
ctagctccaa ctgcctatta t                                                  21

<210>  3
<211>  21
<212>  DNA
<213>  Mus musculus

<400>  3
cctaatccga cagcgaataa a                                                  21

<210>  4
<211>  21
<212>  DNA
<213>  Mus musculus

<400>  4
atttgtgcca aatccatata t                                                  21

<210>  5
<211>  21
<212>  DNA
<213>  Unknown

<220>
<223>  artificial sequence

<400>  5
caacaagatg aagagcacca a                                                  21

<210>  6
<211>  42
<212>  RNA

<213>  Homo sapiens

<400>  6
agaaauaugc cuuuuugucc agacaaaaag gcauauuucu ug                          42


<210>  7
<211>  42
<212>  RNA
<213>  Homo sapiens

<400>  7
acuguuucca ugaaaacccu gggguuuuca uggaaacagu ga                          42


<210>  8
<211>  42
<212>  RNA
<213>  Homo sapiens

<400>  8
uuacuucacu guuuccauga acauggaaac agugaaguaa au                          42


<210>  9
<211>  25
<212>  DNA
<213>  Homo sapiens

<400>  9
caccgaggcc ttggtgtgag catgg                                             25


<210>  10
<211>  25
<212>  DNA
<213>  Homo sapiens

<400>  10
aaacccatgc tcacaccaag gcctc                                             25


<210>  11
<211>  26
<212>  DNA
<213>  Homo sapiens

<400>  11
caccgagttc ctctcctgct gataaa                                            26


<210>  12
<211>  26
<212>  DNA
<213>  Homo sapiens

<400>  12
aaactttatc agcaggagag gaactc                                            26


<210>  13
<211>  25
<212>  DNA

<213>  Homo sapiens

<400>  13
caccggacaa aaaggcatat ttctt                                          25


<210>  14
<211>  25
<212>  DNA
<213>  Homo sapiens

<400>  14
aaacaagaaa tatgcctttt tgtcc                                          25


<210>  15
<211>  36
<212>  DNA
<213>  Homo sapiens


<220>
<221>  misc_feature
<222>  (16)..(16)
<223>  spacer

<400>  15
tgagctgcag ttaacntcag aggactaatg cactaa                              36


<210>  16
<211>  31
<212>  DNA
<213>  Homo sapiens


<220>
<221>  misc_feature
<222>  (16)..(16)
<223>  spacer

<400>  16
tgcagttaac tgttgntcag aggactaatg c                                   31


<210>  17
<211>  33
<212>  DNA
<213>  Homo sapiens


<220>
<221>  misc_feature
<222>  (16)..(16)
<223>  spacer

<400>  17
tgctgctgtt gagctntgca ctaacagttc aac                                 33


<210>  18
<211>  35
<212>  DNA

<213>  Homo sapiens

<220>
<221>  misc_feature
<222>  (16)..(16)
<223>  spacer

<400>  18
tatgtggctg ctgagntcaa cagcagcaat atgca                                35

<210>  19
<211>  35
<212>  DNA
<213>  Homo sapiens

<220>
<221>  misc_feature
<222>  (19)..(19)
<223>  spacer

<400>  19
tcaaaagcac tcccaagant aggtttactc tgatt                                35

<210>  20
<211>  37
<212>  DNA
<213>  Homo sapiens

<220>
<221>  misc_feature
<222>  (17)..(17)
<223>  spacer

<400>  20
tgagaacgta tgaagantag cagcagctca gcagcca                              37

<210>  21
<211>  26
<212>  DNA
<213>  Unknown

<220>
<223>  artificial sequence

<400>  21
ttttctagaa ggtaacatca gccctc                                          26

<210>  22
<211>  36
<212>  DNA
<213>  Unknown

<220>
<223>  artificial sequence

<400>  22

ttttctagat tctttggttg acagtttatt aaatac                                36

<210>  23
<211>  36
<212>  DNA
<213>  Homo sapiens

<400>  23
tttgcatgtt ttgtaaatag gctccagttt tgtttt                                36

<210>  24
<211>  36
<212>  DNA
<213>  Pan troglodytes

<400>  24
tttgcatgtt ttgtaaatag gctccagttt tgtttt                                36

<210>  25
<211>  36
<212>  DNA
<213>  Hylobates agilis

<400>  25
tttgcatgtt ttgtaaatag gctccagttt tgtttt                                36

<210>  26
<211>  36
<212>  DNA
<213>  Callithrix jacchus

<400>  26
tttgcatgtt ttgtaaatag gctccagtgt ggtttt                                36

<210>  27
<211>  36
<212>  DNA
<213>  Oryctolagus cuniculus

<400>  27
tttgcatgtt ttgtaaatag gctcaagtct tttttt                                36

<210>  28
<211>  36
<212>  DNA
<213>  Mus musculus

<400>  28
tttgcatgtc ttgtaaatag gctctaggtt tgtttt                                36

<210>  29
<211>  35
<212>  DNA
<213>  Caenorhabditis elegans

<400>  29

ttagctttat tgtaaatatg ataatagtgt gaatt 35

## Description

### FIELD OF THE INVENTION

The invention relates generally to methods, agents and pharmaceutical compositions for treating or preventing an aging-related disease by reducing the levels and/or activity of the RNA-binding protein PUM2.

### BACKGROUND OF THE INVENTION

Aging is characterized by a functional decline in most biological processes including alterations in cellular (Houtkooper et al., 2010) and mitochondrial metabolism (Sun et al., 2016). Despite the wealth of data available on the transcriptional control of the aging process across different species, the involvement of post-transcriptional regulatory mechanisms is much less studied. Among such processes, regulation of protein synthesis and degradation, which assures proteostasis, is emerging as a key process in aging (Gonskikh and Polacek, 2017; Steffen and Dillin, 2016). RNA-binding proteins (RBPs) are interesting within this context, as they bind ribonucleic-acid molecules regulating their fate after transcription (Shi and Barna, 2015). RBPs contain low-complexity (LC) domains that mediate their condensation in ribonucleoprotein (RNP) granules, which exert a role in mRNA translation, metabolism and transport (Sheinberger and Shav-Tal, 2017). These structures also mediate the response to cellular stress, by assembling RBPs and their target mRNAs in stress granules (SGs), where protein translation is repressed (Sheinberger and Shav-Tal, 2017). The abnormal localization and aggregation of wild-type and mutated forms of RBPs is the cause of several neuromuscular degenerative disorders (Huang et al., 2010; Harrison and Shorter, 2017; Conlon and Manley, 2017). A few relevant examples include the RBPs, FUS and TDP-43, in the context of amyotrophic lateral sclerosis (ALS) and frontotemporal lobar degeneration with ubiquitin-positive inclusions (FTLD-U) (Gitler and Shorter, 2011), PABPN1 in the oculopharyngeal muscular dystrophy (OPMD) (Banerjee et al., 2013) and TIA1 in Tau-dependent Alzheimer's disease (Apicco et al., 2018) and in distal myopathies (Lee et al., 2018). The mechanisms by which the RBPs cause these pathologies are disease-specific. However, a common effect of pathogenic RBPs is a profound alteration of cellular proteostasis, as they lead to the abnormal accumulation and aggregation of RNP granules. In addition, they can impair protein homeostasis at the level of protein synthesis, by affecting the translational efficacy of their target mRNAs.
While the role of RBPs is getting unveiled in the setting of diseases, only a few RBPs were investigated in the context of aging, and the mechanisms underpinning their function in aging are not well characterized (Chaturvedi et al., 2015; Masuda et al., 2009). Towards this end, we systematically screened for RBPs that are altered upon aging of muscle and brain, the tissues in which this class of proteins is most frequently associated with diseases. This led us to identify the RBP, PUM2, as a negative regulator of longevity and healthspan in nematode and mice models and to show that PUM2 in aged tissues leads to accumulation of granules, similarly to other pathogenic RBPs.

PUM2 therefore represents a driver of a novel post-transcriptional mechanism that could link altered proteostasis to mitochondrial dysfunction during aging. Candidate therapeutic agents that reduce the levels and/or activity of the RNA-binding protein PUM2 can thus treat or prevent aging-related diseases.

### DESCRIPTION OF THE FIGURES

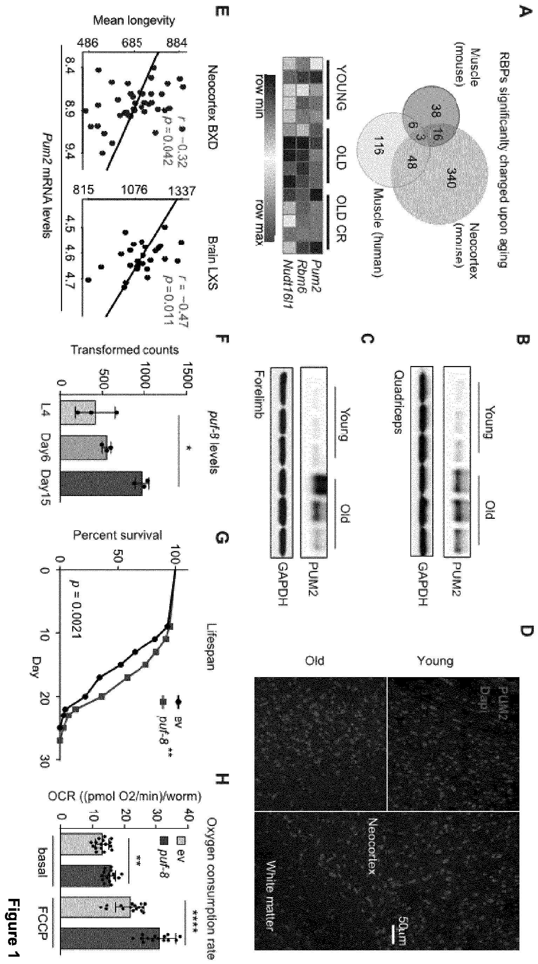

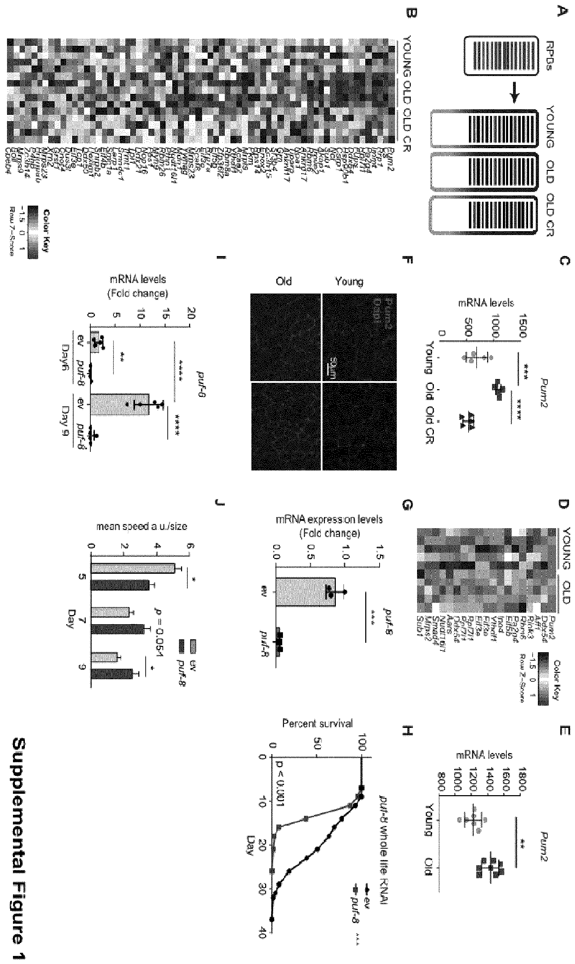

**Figure 1****| Pumilio2 expression is up-regulated upon aging.**
   (A) Top, Venn diagram representing common significantly changed transcripts amongst 1343 different RBPs in old *vs.* young and old caloric restricted *vs.* old mouse gastrocnemius muscle (from GSE6323, p < 0.05), young *vs.* old mouse neocortex tissues (from GSE13120, p< 0.001) and muscle biopsies from aged humans (Yang et al., 2015). The expression of the 3 common hits, *Pum2, Rbm6* and *Nudt16l1,* in young, old and old caloric restricted gastrocnemius tissues (GSE6323) is shown in the heatmap below. (B and C) Western blot of PUM2 and GAPDH as loading control in young (2 month) and old (24 month) quadriceps (B) and forelimb (C) muscles from C57BL/6J mice. (D) Immunostaining of PUM2 (red) in neocortex tissues from young (2 month) and old (24 month) C57BL/6J mice. Nuclei are counterstained in blue with Hoechst. Scale bar = 50um. (E) Correlation analysis of *Pum2* mRNA levels (x axis) in neocortex from the BXD (left) and whole brain tissues from the LXS (right) mouse genetic reference populations, respectively, and mean longevity (y axis). Coefficient of correlation (*r*) and p value (*p*) are shown in red. (F) mRNA levels of *puf-8,* the *C*. *elegans* orthologue of *Pum2,* in N2 worms at the larval stage (L4), adult (Day 6) and old worms (Day 15). Data expressed as transformed counts, from GSE21784. •p < 0.05, unpaired two-tailed Student's t test. Error bars represent the mean ± SD. (G) Lifespan curves of *glp-1(e2144)* worms fed with empty vector (ev, black) or *puf-8* RNAi (*puf-8,* red), from Day 1 of adulthood. ••p < 0.05, log-rank (Mantel-Cox) test. (H) Oxygen consumption rates showing basal and FCCP-induced maximal respiration in *glp-1* worms fed as in Figure 1F. ••p < 0.01; ••••p < 0.0001, unpaired two-tailed Student's t test. Error bars represent the mean ± SD.
      See also Figure S1.

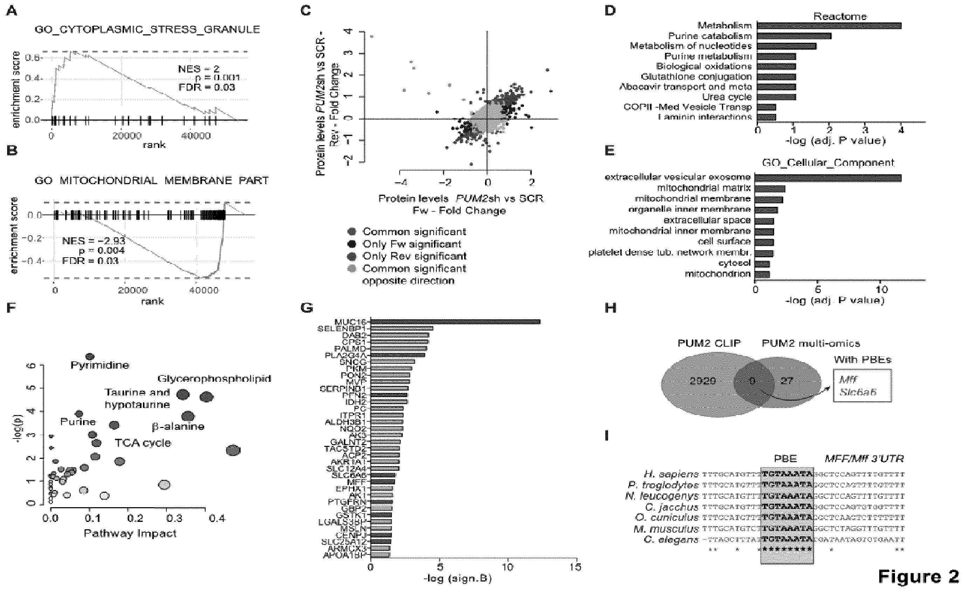

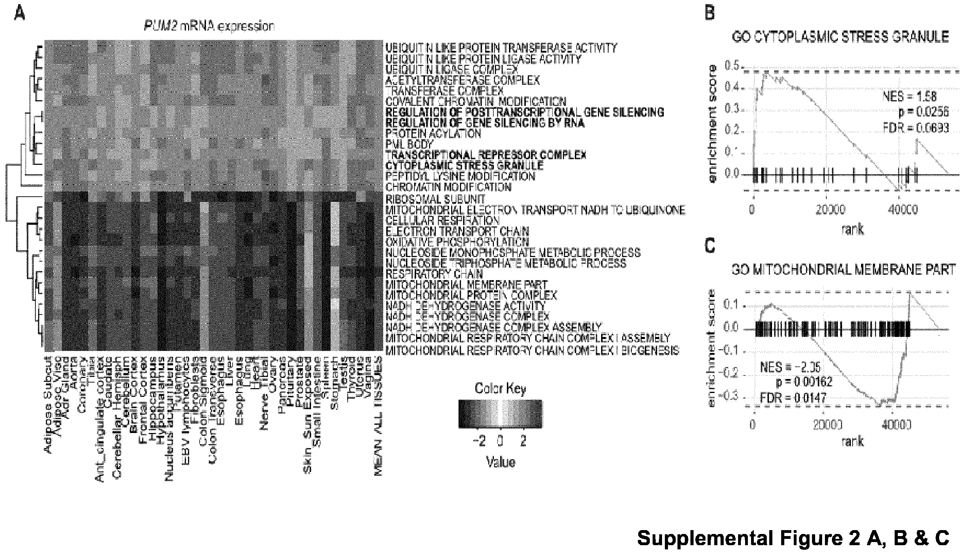

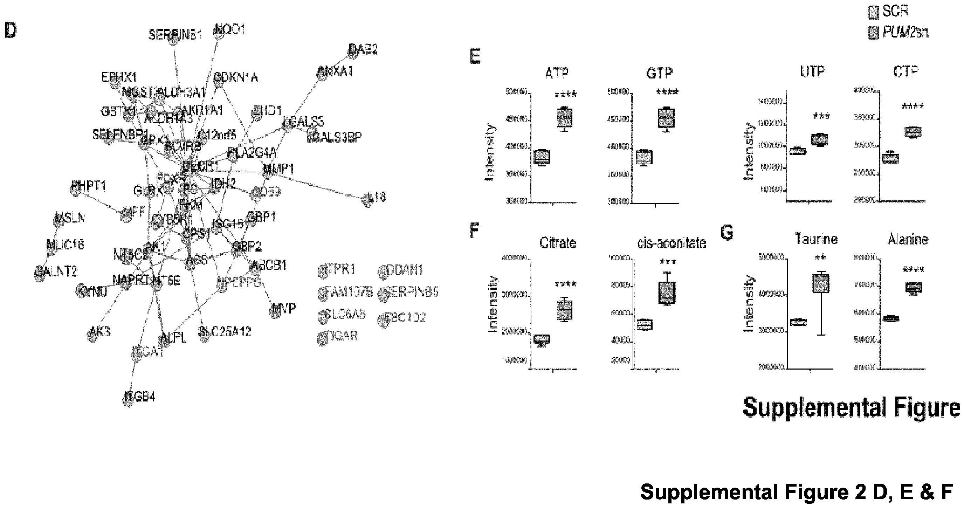

**Figure 2** **| Multi-omics analyses reveal PUM2 as negative regulator of mitochondrial function.**
   (A and B) Gene set enrichment analysis of *PUM2* in human cortex indicates its positive correlation with the gene ontology (GO) category "Cytoplasmic stress granule" (A) and negative correlation with the category "Mitochondrial membrane part" (B). NES, normalized enrichment score. FDR, false discovery rate. (C) Analysis of proteomic changes in HeLa cells stably expressing either a scramble (SCR) or a *PUM2*-targeting short-hairpin sequence *(PUM2*sh*).* Scatterplot depicts SILAC Forward (Fw, x-axis) and Reverse (Rev, y-axis) replicates comparing *PUM2*sh *vs.* SCR cells. Each dot represents a single protein level, expressed as fold-change. In red are the proteins significantly regulated in both replicates (sign.B < 0.05) and with common direction of their fold change. Supplementary Table 1 contains the full proteomics dataset. (D and E) Pathway enrichment analysis for the Reactome (C) and GO cellular-component categories (D) of the 85 commonly induced proteins upon *PUM2*sh in HeLa stable clones. (F) Metabolome pathway enrichment analysis of up-regulated metabolites in *PUM2*sh vs SCR HeLa cells. Scatter-plot represents p values from integrated enrichment analysis and impact values from pathway topology analysis. The node color is based on the p values and the node radius represents the pathway impact values. (G) Ranking of the 36 proteins that, upon *PUM2*sh*,* are significantly upregulated (sign.B < 0.05 SILAC Fw), but whose mRNA levels are not significantly changed (adj.p val > 0.05) or downregulated (so with opposite direction in respect of the protein levels) (adj.p.val < 0.05; log(Fold change) < 0). X-axis represent the -log(sign.B) for each hit. The 9 common hits from Figure 2H are highlighted in red. (H) Venn diagram representing mRNAs bound by PUM2 (PUM2 CLIP, from(Hafner et al., 2010)) and the 36 proteins from Figure 2G. Among the 9 common hits, MFF and SLC6A6 are the only proteins whose corresponding mRNAs contain at least one Pumilio-binding element (PBE).
      (I) Alignment of the *MFF*/*Mff* 3'UTR regions containing the PBE in the indicated species. The square highlights the evolutionary conservation of the PBE sequence. Asterisks indicate positions with fully conserved residues.

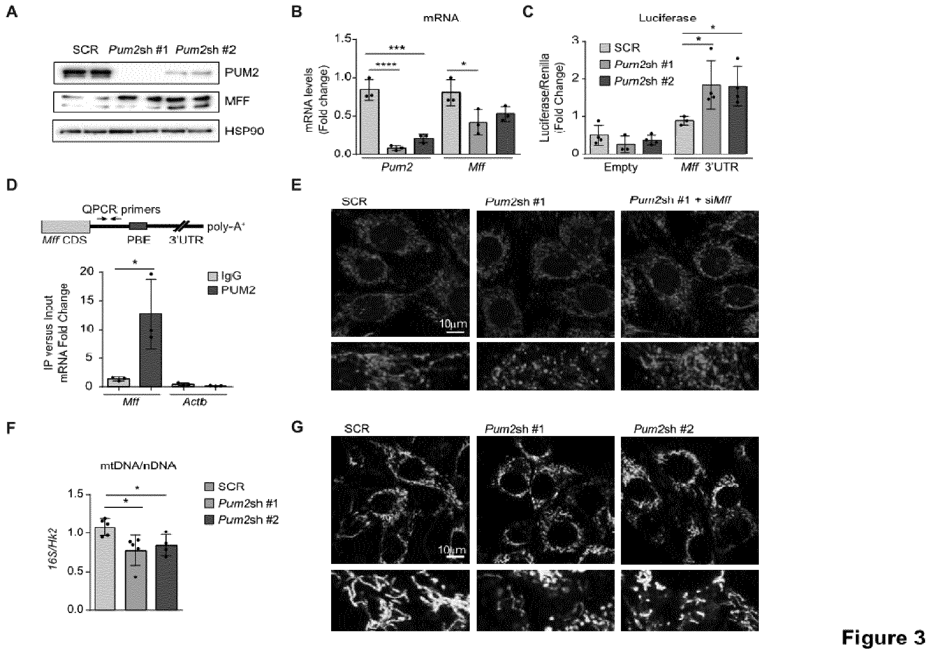

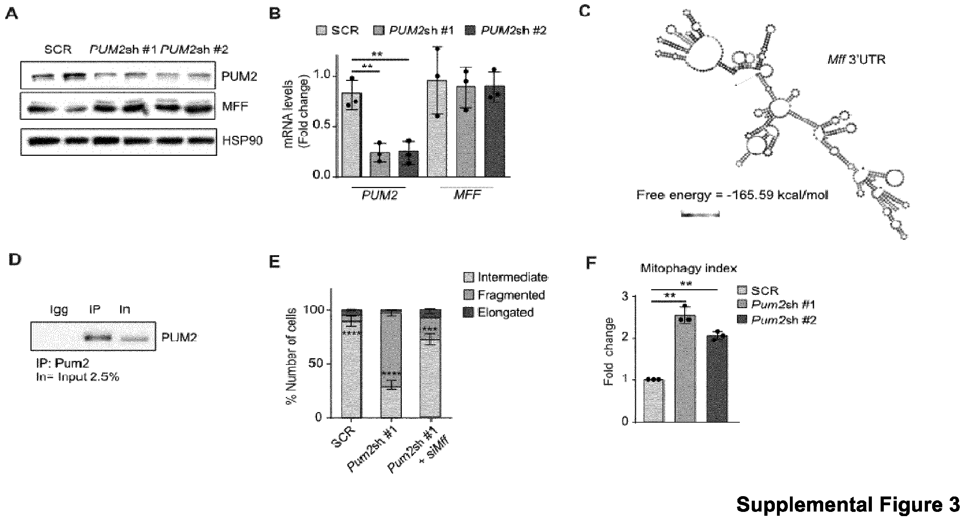

**Figure 3** **| PUM2 regulates mitochondrial fission and mitophagy via MFF.**
   Analysis of mouse myoblast C2C12 stable cell clones, expressing a control shRNA (SCR, grey) or two independent *Pum2*-targeting shRNAs (*Pum2*sh #1 in orange, *Pum2*sh #2 in red). (A) Protein levels of PUM2, MFF and HSP90 (as loading control) in C2C12 cells. (B) Quantitative-RT-PCR (q-RT-PCR) of *Pum2* and *Mff* normalized to *Gapdh* mRNA levels. Data are expressed as fold change (n=3). •p < 0.05; •••p < 0.001, ••••p < 0.0001, one-way ANOVA. Error bars represent the mean ± SD.
   (C) Luciferase assay in C2C12 SCR, *Pum2*sh #1 and *Pum2*sh #2 stable clones expressing the pGL3-Promoter vector containing either the *Mff* 3'UTR or no additional sequences (Empty), downstream to the Luciferase ORF. C2C12 cells were co-transfected with the pRL-CMV Renilla-expressing plasmid. Values represent the ratio of Luciferase to Renilla signals, expressed as fold change (n=3). •p < 0.05, unpaired two-tailed Student's t test. Error bars represent the mean ± SD.
   (D) Top, representative scheme of the qPCR strategy after cross-linking and immunoprecipitation (CLIP) of PUM2 or IgG (as control). In brief, after immunoprecipitation, IgG- and PUM2-bound mRNAs were eluted, retro-transcribed and the corresponding cDNA was analysed for *Mff* and *Actb* levels (bottom). An aliquot of the IP'ed samples was analysed to assess immunoprecipitation efficiency (Figure S3D). Values represent the ratio of the mRNA levels in the IP'ed fraction normalized by the levels in the corresponding total mRNA fraction. Values are expressed as fold change (n=3). •p < 0.05, unpaired two-tailed Student's t test. Error bars represent the mean ± SD. (E) Confocal immunofluorescence imaging of SCR, *Pum2*sh #1, and *Pum2*sh #1 C2C12 cells. In some cells, *Mff* was knocked-down by small-interfering RNA (si*Mff*). Mitochondria are stained for Tom20 (green) and nuclei are shown in blue after DAPI staining. Bottom, higher magnification of the mitochondrial networks. Scale bar = 10 um. Quantification of the % of cells undergoing fission is shown in Figure S3E.
   (F) Quantification of the mtDNA/nDNA ratio in the indicated C2C12 clones using the *16S* and Hexokinase 2 (*Hk2*) loci, respectively. Values are expressed as fold change (n=5). •p < 0.05, one-way ANOVA. Error bars represent the mean ± SD.
   (G) C2C12 cell clones expressing SCR, *Pum2*sh #1 and *Pum2*sh #2 analysed with the m-KEIMA fluorophore showing increased mitophagy (red signal). Scale bar = 10 um. Quantification of the mitophagic rate is shown in Figure S3F.
      See also Figure S3.

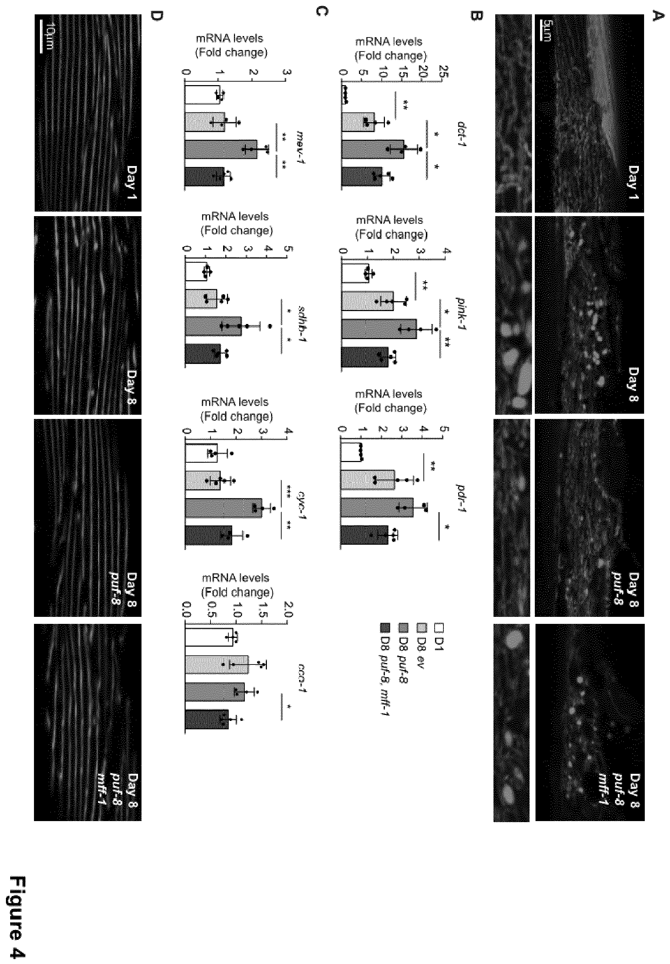

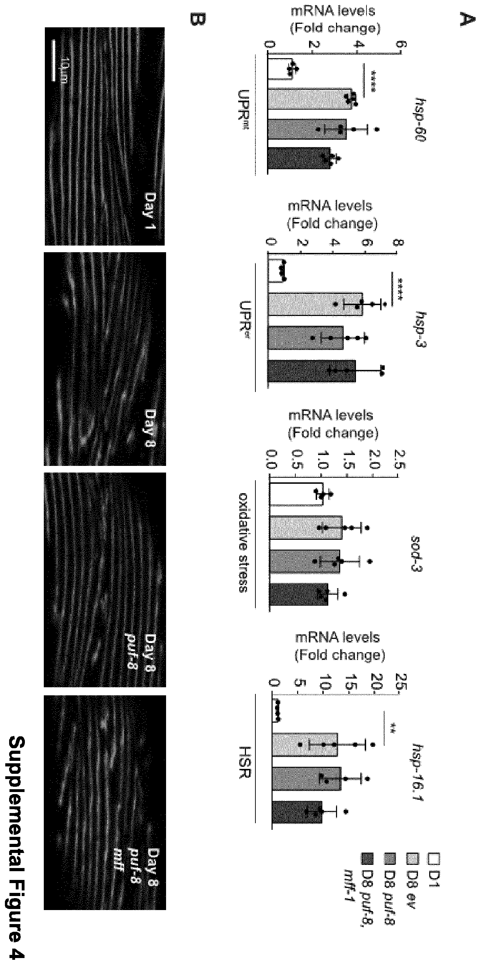

**Figure 4** **| *puf-8* regulates mitochondrial fission *in vivo* in aged worms.**
   (A) Confocal imaging of worms expressing the muscle-specific p_{myo-3}::mito::GFP reporter to visualize mitochondrial morphology. Images compare young worms (Day1) fed with control RNAi and old worms fed with either control RNAi (Day 8) or with *puf-8* RNAi (Day 8, *puf-8*) or with both *puf-8* and *mff-1* RNAi (Day 8, *puf-8 mff-1*). Bottom, higher magnification of the mitochondrial networks. Scale bar = 5 um. (B and C) q-RT-PCR analysis of the mitophagy marker genes *dct-1, pink-1* and *pdr-1* (B) and the OXPHOS genes *mev-1, sdhb-1, cyc-1* and *cco-1* (C), normalized to the levels of *pmp-3* and *act-1,* in *glp-1* worms treated as in Figure 4A (n>4). •p < 0.05; ••p < 0.01; •••p < 0.001, unpaired Student's t test. Error bars represent the mean ± SD. (D) Confocal imaging of N2 worms expressing myo-3::GFP, to visualize muscle fibers, in worms treated as in Figure 4A. Scale bar = 10 um. See also Figure S4B.
      See also Figure S4.

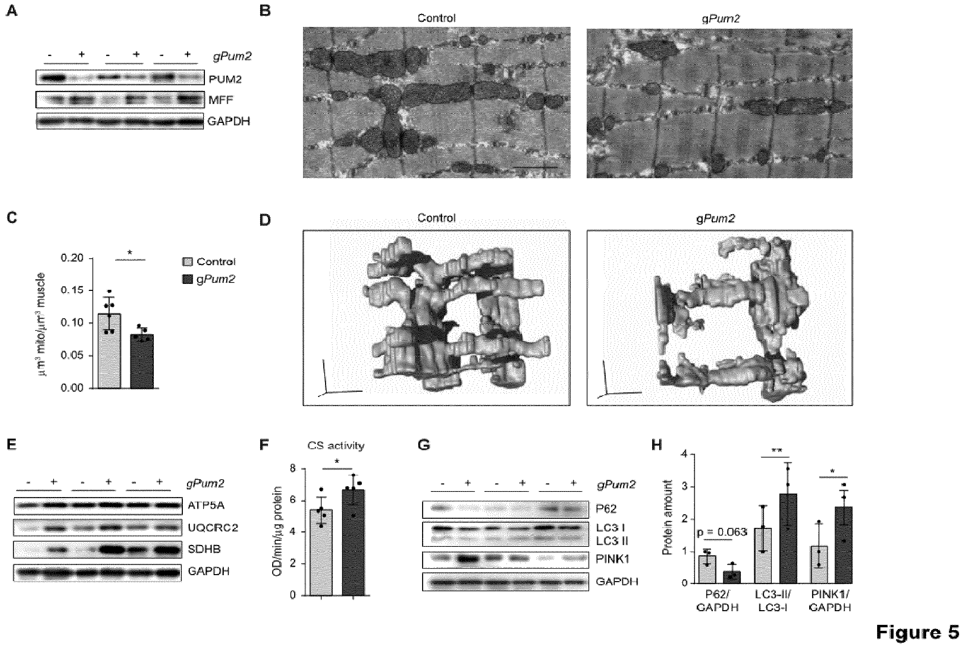

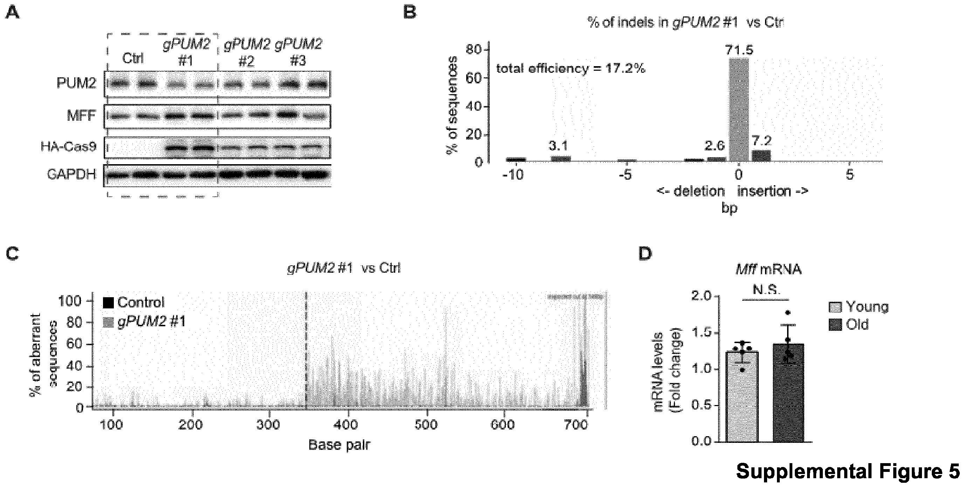

**Figure 5** **| PUM2 regulates mitochondrial function in old mice.**
   (A) Western blot of PUM2 and MFF (GAPDH levels as loading control) in quadriceps muscles of old mice (24 months) injected either with AAV9s containing only Cas9 as control (-) or both Cas9 and a *Pum2*-targeting gRNA (+ g*Pum2*). (B) Representative electron micrographs showing the morphology of intermyofibrillar (IFM) mitochondria of control and g*Pum2* injected mouse gastrocnemius muscles analysed by transmission electron microscopy. Scale bar = 1 um. (C and D) Mitochondrial volume quantification expressed as um³ of mitochondria per um³ of muscle (n = 6) (C) and representative 3D reconstructions (D) of mitochondria from mice muscles as in Fig. B. Scale bar = 0.5 uM. •p < 0.05, unpaired two-tailed Student's t test. Error bars represent the mean ± SD. (E and F) Western blot analysis of the OXPHOS components ATP5A, UQCRC2 and SDHB (GAPDH as loading control) (E) and citrate synthase activity (F) in old quadriceps muscle tissues as in Figure 5A (n = 5). •p < 0.05, ratio paired Student's t test. Error bars represent the mean ± SD.
   (G and H) Western blot (G) and quantification (H) of PINK1, LC3-I/LC3-II, P62 and GAPDH in old quadriceps muscle tissues as in Figure 5A. •p < 0.05; ••p < 0.01, ratio paired Student's t test. Error bars represent the mean ± SD.
      See also Figure S5.

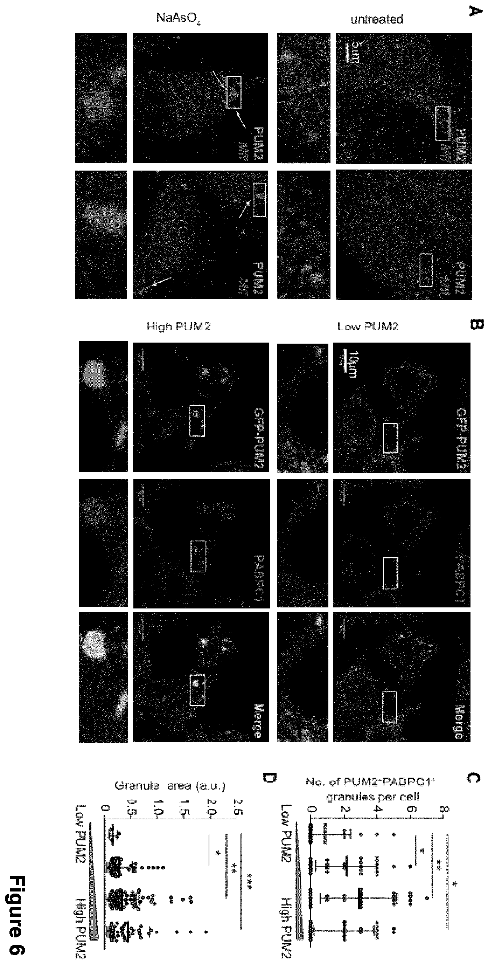

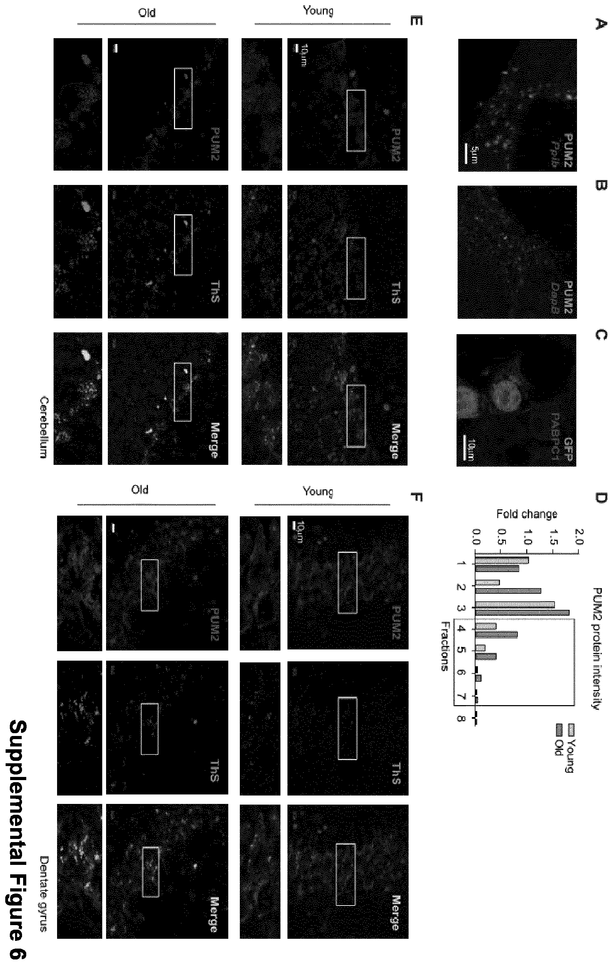

**Figure 6** **| PUM2 forms *Mff*-containing stress granules.**
   (A) Representative confocal images of C2C12 myoblasts untreated or treated with sodium arsenite (NaAsO₂, 0.5mM, 45 min) and stained with an antibody detecting the endogenous PUM2 protein (green) and with a fluorescent probe targeting *Mff* mRNA (red). Arrows indicate SGs associated with *Mff* mRNA. Scale bar = 5 um. Bottom, higher magnification. (B) Representative confocal images of HEK239T cells transfected with increasing amounts of GFP-PUM2 (Low = 0.03ug, high =1ug in sub-confluent cells seeded in 6 well plates). Cells were then stained for endogenous PABPC1 (red). See also Figures S6A and B. (C and D) Corresponding quantification of the number of GFP-PUM2 granules colocalizing with PABCP1 (C) and of the size of GFP-PUM2 granules (D) (the two intermediate conditions, comprised between Low and High, were transfected with 0.1ug and 0.3ug of GFP-PUM2, respectively) (n>22). Bottom, higher magnification. Scale bar = 10 um. See also Figure S6C. •p < 0.05; ••p < 0.01; •••p < 0.001, unpaired Student's t test. Error bars represent the mean ± SD.
      See also Figure S6.

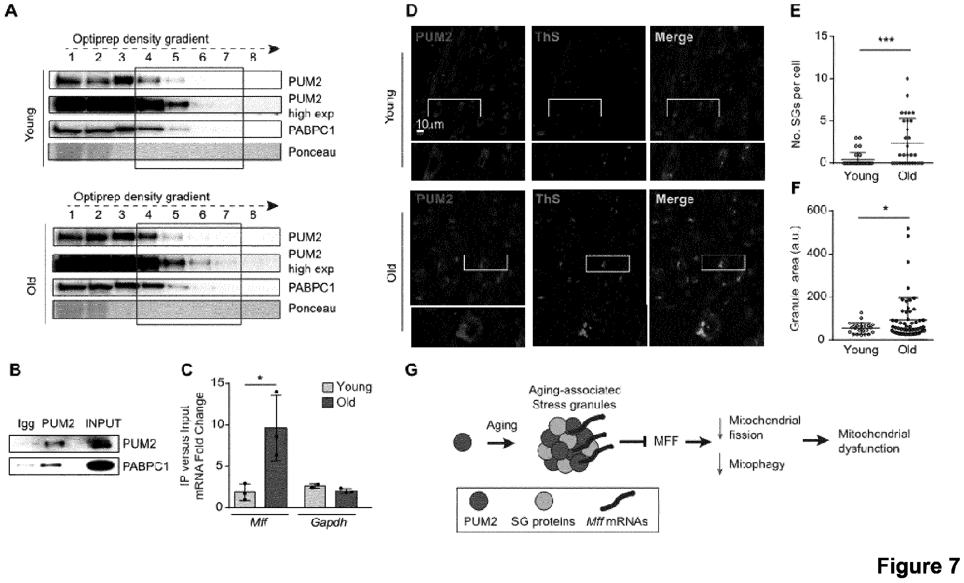

**Figure 7** **| PUM2 forms *Mff*-containing aging-associated stress granules *in vivo.***
   (A) Size fractionation of RNP granules using the Optiprep density gradient centrifugation of whole protein extract from brains of young (top; 2 month) or old (bottom; 24 month) C57BL/6J mice. Filters were stained with Ponceau S to assess the fractionation of the whole pool of cellular proteins and then probed with the indicated antibodies. PUM2 and PABPC1 cofractionate in the heavy fractions (4-7). See also Figure S6D. (B) PUM2 co-immunoprecipitation assay from the pooled fractions 4 to 7 from old tissue from Figure 7A. Equal volumes of each fraction were mixed and the lysate divided in two aliquots, one was incubated with control IgG and the other with an antibody targeting PUM2. Western blot analysis shows co-immunoprecipitation of PUM2 and the SG component, PABPC1. Input, 1% of total lysate. (C) q-RT-PCR of the mRNA bound to endogenous PUM2 IP'ed from old brain tissue, as in Figure 7B. RNA was extracted from the IP'ed samples, retro-transcribed and the corresponding cDNA was amplified with primers encompassing *Mff* 3'UTR or *Gapdh,* as control. (D) Co-Immunostaining of PUM2 (red) and ThioflavinS (ThS, green) in the neocortex of young (2 month; top) and old (24 month, bottom) mouse brains. Nuclei are counterstained in blue with Hoechst. Bottom, higher magnification. Scale bar = 10 um. (E and F) Corresponding quantification of number (E, n>21) and size (F, n=6 Low, n>43 for the other conditions) of PUM2 granules. •p < 0.05, unpaired Student's t test, •••p < 0.001, unpaired Student's t test. Error bars represent the mean ± SD. (G) Schematic representation of a model of the PUM2-containing aging-associated SG. PUM2 (red circles) increases upon aging and interacts with other SG proteins, such as PABPC1 (yellow circles). PUM2 binds and sequesters *Mff* mRNA in these aging SGs preventing its translation and impairing mitochondrial fission, mitophagy and consequently mitochondrial function.
      See also Figure S6.
**Figure S1 | Pumilio2 expression is up-regulated upon aging in worm and mouse models and humans, Related to** **Figure 1****.**
   (A) Workflow to identify significantly regulated RNA-binding-proteins (RBPs, red lines) during aging. (B) Heatmap of the 66 significantly changed transcripts amongst 1343 different RBPs in old *vs.* young and old CR *vs.* old mouse muscle in the GSE6323 dataset (p< 0.05). (C) mRNA expression of *Pum2* in the GSE6323 dataset (n=5). •••p < 0.001; ••••p < 0.0001, unpaired two-tailed Student's t test. Error bars represent mean ± SD (D) Heatmap of the 19 RBP transcripts significantly changed in the mouse neocortex aging dataset GSE13120 (p< 0.01) in common with the RBPs in Figure S1B. (E) mRNA expression levels of *Pum2* in the mouse neocortex aging dataset GSE13120 (n=5). ••p < 0.01, unpaired two-tailed Student's t test. Error bars represent mean ± SD (F) Immunostaining of PUM2 in tibialis muscles from young (2 month) and old (24 month) C57BL/6J mice (n = 2). Scale bar = 50 um. (G) mRNA levels of *puf-8* in WT N2 young worms (Day 1) fed, from hatching with either control (ev, black) or an RNAi targeting *puf-8* (*puf-8,* red). Values are normalized to *pmp-3* and *act-1* and expressed as fold change (n=3). •••p < 0.001, unpaired two-tailed Student's t test. Error bars represent mean ± SD. (H) Lifespan curve of N2 worms fed with empty vector (ev, black) or *puf-8* RNAi (*puf-8,* red) from hatching. •••p < 0.001, log-rank (Mantel-Cox) test. (I) mRNA levels of *puf-8* in *glp-1* adult (Day 6) and old (Day 9) worms fed, from Day 1 of adulthood with either control (ev) or *puf-8* targeting RNAi (*puf-8*). Data show the age-dependent increase of *puf-8* levels and the efficiency of the RNAi at both time points. Values are normalized to *pmp-*3 and *act-1* and expressed as fold change (n=5). ••••p < 0.0001, unpaired two-tailed Student's t test. Error bars represent mean ± SD. (J) Mobility at the indicated days of adulthood of *glp-1* worms treated with ev or puf-8 RNAi from Day 1 (n>32 Day5, n>28 Day 7, n>19 Day9). •p < 0.05, unpaired Student's t test. Error bars represent mean ± SEM.
**Figure S2 | Multi-omics analyses reveal PUM2 as negative regulator of mitochondrial function, Related to** **Figure 2****.**
   (A) Heat-map showing the top gene sets enriched for the up-regulation of PUM2 expression using GSEA in human transcriptome data from GTEx cohort. The corresponding tissues of the transcriptome data were indicated. GO-terms to which *PUM2* is known to belong are in bold. NES = Normalized enrichment score. (B and C) Enrichment plot of PUM2 in human frontal cortex samples indicates its positive correlation with the GO category "Cytoplasmic stress granule" (B) and negative correlation with the category "Mitochondrial membrane part" (C). NES, normalized enrichment score. FDR, false discovery rate. (D) STRING Interaction network analysis of the 85 upregulated protein from Figure 2C (interaction score > 0.500). Proteins containing at least one Pumilio Binding Element (PBE) are in red. Proteins not connected to the network are not shown, except if they contain PBEs. (E and G) Box-whisker plots representing the levels of the nucleotides ATP, GTP, UTP and CTP (E), of the TCA components, citrate and cis-aconitate (F) and of the metabolites, alanine and taurine (G) in SCR (grey) and PUM2sh (orange) HeLa stable cells. Values are expressed as peak intensity. Full metabolome is described in Supplementary Table 3. ••p < 0.01; •••p < 0.001; ••••p < 0.0001, unpaired two-tailed Student's t test. Error bars represent mean ± mean to max.
**Figure S3** | **PUM2 regulates mitochondrial fission and mitophagy via MFF, Related to** **Figure 3****.**
   Analysis of human HeLa stable cell clones, expressing a control shRNA (SCR, grey) or two independent *PUM2*-targeting shRNAs (*Pum2*sh #1 in orange, *Pum2*sh #2 in red). (A) Protein levels of PUM2, MFF and HSP90 (as loading control). (B) Quantitative-RT-PCR (q-RT-PCR) of *PUM2* and *MFF* normalized to *GAPDH* mRNA levels. Data are expressed as fold change (n=3). ••p < 0.01, one-way ANOVA. Error bars represent mean ± SD. (C) Secondary structure of the mouse *Mff* 3'UTR sequence, calculated by Vienna RNAfold according to the minimum free energy "MFE" criteria. Free energy of the structure is expressed as kcal/mol. (D) Immunoprecipitation efficiency of PUM2 related to the CLIP-q-RT-PCR shown in Figure 2D. (E) Quantification of the changes in mitochondrial morphology from Figure 3E. Values represent the percentage of cells with indicated mitochondrial morphologies from three independent experiments (cell no. per experiment > 40) as defined in(Otera et al., 2010). •••p < 0.001; ••••p < 0.0001, comparing intermediate vs. fragmented with unpaired two-tailed Student's t test. Error bars represent mean ± SEM. (F) Mitophagic index of cells in Figure 3G, calculated as in (Zheng et al., 2017). Graph represents three independent experiments. ••p < 0.01; •••p < 0.001, unpaired two-tailed Student's t test. Error bars represent mean ± SEM.
**Figure S4 | *puf-8* regulates muscle homeostasis in aged worms without affecting stress-related pathways, Related to** **Figure 4****.**
   (A) q-RT-PCR analysis of *hsp-60* as a marker of the mitochondrial unfolded protein response (UPR^{mt}), *hsp-3* as a marker of the endoplasmic reticulum unfolded protein response (UPR^{er}), *sod-3* as a marker of oxidative stress and *hsp-16.1* as a marker of the heat-shock response (HSR). All transcript levels are normalized to the levels of the housekeeping genes, *pmp-3* and *act-1* (n>4). ••p < 0.01; ••••p < 0.0001, unpaired Student's t test. Error bars represent mean ± SD. (B) Confocal imaging of N2 worms expressing myo-3::GFP, to visualize muscle fibers, in worms treated as in Figure S4A. Bottom, higher magnification. Scale bar = 10 um.
**Figure S5** | **Selection of the most efficient guide RNA to knock-down *Pum2* and induce protein, but not mRNA, levels of MFF, Related to** **Figure 5****.**
   (A) Western blot analysis to assess the efficiency of three plasmids encoding different guide RNAs (gRNAs) targeting PUM2 (g*PUM2* #1, *gPUM2* #2, *gPUM2* #3), compared to the control vector (Ctrl, expressing Cas9 only), in 293T cells. Plasmid containing the *gPUM2* #1, which is most efficient in knocking-down PUM2 expression and inducing its target MFF, was selected to produce the AAV9s used for the *in vivo* mouse experiments in Figure 5 (referred as g*Pum2*). All the g*PUM* sequences target both human and mouse genomes. Filters are probed for PUM2 and MFF. The Cas9 expressed in these plasmids is fused with an HA-tag, which is used to verify its expression. GAPDH is used as loading control. (B and C) Percentage of insertions-deletions (B, total indel efficiency = 17.2%) and of aberrant sequences (C) around and downstream of the genomic locus targeted by *gPUM2* #1 (represented as bp = 0 for B and as a dotted blue line in C) compared to the gDNA. Results are from the HEK293T cells in Figure S5A transfected with either *gPUM2* #1 or the control vector. (D) mRNA levels of *Mff* in young and old mice from Figure 5A, normalized to *B2m* mRNA levels (n=5). N.S. = Not Significant.
**Figure S6 | PUM2 forms *Mff*-containing aging-associated stress granules *in vitro* and *in vivo,* Related to** **Figure 6** **and** **Figure 7****.**
   (A and B) C2C12 cells stained for PUM2 protein (green) and a probe targeting either the *Ppib* mRNA (encoding for the Cyclophilin B protein) as positive control (A), or the bacterial *DapB* mRNA, used as negative control (B). Scale bar = 5 um. (C) Representative image of 293T cells transfected with "High" amount (1ug) of a GFP-only expressing vector. No aggregation of the GFP is observed. Scale bar = 10 um. (D) Quantitation of the relative PUM2 protein levels in the gradients from Fig. 7A. (E and F) Co-Immunostaining of PUM2 (red) and ThioflavinS (ThS, green) in cerebellum (E) and dentate gyrus (F) of young (2 month) and old (24 month) mice, as in Fig.7D. Nuclei are counterstained in blue with Hoechst. Bottom, higher magnification. Scale bar = 10 um.

### SUMMARY OF THE INVENTION

The present invention provides a method of treating or preventing an aging-related disease, the method comprising administering to a subject in need thereof a pharmaceutical composition comprising a therapeutic or prophylactic amount of at least one agent that reduces the levels and/or activity of the RNA-binding protein PUM2.

A further object of the present invention is to provide a pharmaceutical composition comprising at least one agent that reduces the levels and/or activity of the RNA-binding protein PUM2.

A further object of the present invention is to provide a pharmaceutical composition comprising at least one agent that reduces the levels and/or activity of the RNA-binding protein PUM2 for use in treating or preventing an aging-related disease.

A further object of the present invention is to provide a gene delivery vector comprising
i) a Cas9 endonuclease, and
ii) at least one single guide RNA (sgRNA), or crRNA and tracrRNA, recognizing a DNA target sequence comprising 16 to 25 nucleotides wherein said DNA target sequence is selected from the group comprising the *Pum2* gene and a regulatory sequence of the *Pum2* gene.

A further object of the present invention is to provide a gene delivery vector comprising i) a nucleic acid encoding at least one TALEN protein, wherein the at least one TALEN protein is capable of inducing a site-specific double stranded DNA break in a DNA target sequence selected from the group comprising the *Pum2* gene and a regulatory sequence of the *Pum2* gene, and ii) at least one nucleic acid donor sequence, wherein the donor sequence is a template for targeting sequence selected from the group comprising the *PUM2* gene and a regulatory sequence of the *PUM2* gene.

A further object of the present invention is to provide a nucleic acid encoding an miRNA, siRNA, piRNA, hnRNA, snRNA, esiRNA, shRNA, or antisense oligonucleotide that reduces the levels and/or activity of the RNA-binding protein PUM2.

A further object of the present invention is to provide a kit comprising i) a pharmaceutical composition of the invention ii) a gene delivery vector of the invention or iii) an acid nucleic of the invention .

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of include/including, that is to say permitting the presence of one or more features or components.

As used in the specification and claims, the singular form "a", "an" and "the" include plural references unless the context clearly dictates otherwise.

As used herein, "at least one" means "one or more", "two or more", "three or more", etc.

As used herein the terms "subject"/"subject in need thereof', or "patient"/"patient in need thereof " are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult and newborn subjects, whether male or female, are intended to be covered.

The terms "nucleic acid", "polynucleotide," and "oligonucleotide" are used interchangeably and refer to any kind of deoxyribonucleotide (e.g. DNA, cDNA, ...) or ribonucleotide (e.g. RNA, mRNA, ...) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are modified in the base, sugar and/or phosphate moieties (e.g. phosphorothioate backbones). In general, an analogue of a particular nucleotide has the same base-pairing specificity; i.e., an analogue of A will base-pair with T.

The term "vector", as used herein, refers to a viral vector or to a nucleic acid (DNA or RNA) molecule such as a plasmid or other vehicle, which contains one or more heterologous nucleic acid sequence(s) (such as, e.g. nucleic acid sequence(s) encoding the sgRNA, TRACR and CrRNA, or the Cas9 nuclease of the invention), and is designed for transfer between different host cells. The terms "expression vector", "gene delivery vector" and "gene therapy vector" refer to any vector that is effective to incorporate and express one or more nucleic acid(s), in a cell, preferably under the regulation of a promoter. A cloning or expression vector may comprise additional elements, for example, regulatory and/or post-transcriptional regulatory elements in addition to a promoter.

Any suitable vector can be employed that is effective for introduction of one or more nucleic acid(s) of the invention into cells. Preferably, the gene delivery vector of the invention is a viral vector or a plasmid vector. More preferably, the gene delivery vector is a viral vector, such as a lenti- or baculo- or even more preferably adeno-viral/adeno-associated viral (AAV) vector, but other means of delivery or vehicles are known (such as yeast systems, microvesicles, gene guns/means of attaching vectors to gold nanoparticles). In some aspects, one or more of the viral or plasmid vectors may be delivered via liposomes, nanoparticles, exosomes, microvesicles, or a gene-gun. Most preferably, the gene delivery viral vector is selected from the group comprising an adeno-associated virus (AAV) and a lentivirus. Lentivirus of 1st, 2nd, and 3rd generation are also envisioned and are known in the art.
The type of AAV surface protein determines the target tissue. Preferably, any adeno -associated virus (AAV) serotype or engineered AAV is envisioned. Most preferably, the AAV will be selected from the group comprising AAV6 and AAV9, due to their broad tissue specificity and expression levels. AAV9 particularly has a minimal inflammatory response, thereby reducing the side effects. The viral particles will usually be administered by injection into the bloodstream. AAV6 can also be used with cultured patient-derived cells as described herein.

The term "about," particularly in reference to a given quantity, is meant to encompass deviations of plus or minus ten (10) percent.

The term "treatment" or "treating" means any administration of a composition, pharmaceutical composition, compound, agent, *etc...* of the disclosure to a subject for the purpose of:
(i) inhibiting the disease, condition, or disorder, that is, arresting the development of clinical symptoms; and/or
(ii) relieving the disease, condition, or disorder, that is, causing the regression of clinical symptoms.

The term "prevention" or "preventing" means any administration of a composition, pharmaceutical composition, compound, agent, *etc...* of the disclosure to a subject for the purpose of preventing the disease, condition, or disorder, that is, causing the clinical symptoms of the disease.
In the context of the present invention, the disease, condition, or disorder is alleviated by treatment with a compound, agent, composition, or pharmaceutical composition, of the disclosure that reduces the levels and/or activity of the RNA-binding protein PUM2. Preferably, said disease, condition, or disorder is an aging-related disease.

Most preferably, said aging-related disease is selected from the non-limiting group comprising muscle diseases, such as e.g., inclusion body myositis, age-related sarcopenia, frailty of the elderly, and muscular dystrophy's (such as Duchenne's or Becker's muscular dystrophy).

In some aspects, the aging-related disease is a neurodegenerative disease. Most preferably, the neurodegenerative disease is selected from the non-limiting group comprising Alzheimer's disease, Dementia, Parkinson's disease, Huntington's disease, and amyotrophic lateral sclerosis.

"Administering" or "administration", as it applies herein, refers to contact of one or more pharmaceutical compositions of the invention, to the subject, preferably the subject in need thereof.

Surprisingly, the inventors of the present invention have shown that genome editing of *Pum2* in the muscle of elderly mice shows that Pumilio2 (PUM2) regulates mitochondrial dynamics and function, as well as mitochondrial clearance upon aging in a similar fashion as in *C*. *elegans.* PUM2, which is a translation repressor, is thus induced upon aging and acts as a negative regulator of lifespan and mitochondrial homeostasis.

The present invention thus concerns a method of treating or preventing an aging-related disease, the method comprising administering to a subject in need thereof a pharmaceutical composition comprising a therapeutic or prophylactic amount of at least one agent that reduces the levels and/or activity of the RNA-binding protein PUM2.
In one aspect, said agent either inhibits the translation of an RNA encoding PUM2 or the transcription of a DNA encoding PUM2 and/or inhibits or impairs the binding of the PUM2 protein to a target RNA.
In the foregoing methods, the agent can be a synthetic compound or a naturally occurring compound. For example, the agent can be selected from the group comprising a chemical agent (e.g. a small molecule), a peptide or analog thereof, an antibody or an antigen-binding fragment of said antibody, a gene delivery vector and a nucleic acid, and combination thereof.

As used herein, an "antibody" is a protein molecule that reacts with a specific antigenic determinant or epitope and belongs to one or five distinct classes based on structural properties: IgA, IgD, IgE, IgG and IgM. The antibody may be a polyclonal (e.g. a polyclonal serum) or a monoclonal antibody, including but not limited to fully assembled antibody, single chain antibody, antibody fragment, and chimeric antibody, humanized antibody as long as these molecules are still biologically active and still bind to at least one peptide of the invention. Preferably the antibody is a monoclonal antibody. Preferably also the monoclonal antibody will be selected from the group comprising the IgG1, IgG2, IgG2a, IgG2b, IgG3 and IgG4 or a combination thereof. Most preferably, the monoclonal antibody is selected from the group comprising the IgG1, IgG2, IgG2a, and IgG2b, or a combination thereof. The antibody of the invention is preferably an anti- PUM2 protein antibody that reduces the levels and/or activity of the RNA-binding protein PUM2 e.g. by inhibiting or impairing the binding of the PUM2 protein to the target RNA. Preferably, said target RNA is an mRNA containing at least one motif 5•-UGUAXAUA-3• known as a Pumilio-binding element (PBE).

A typical antibody is composed of two immunoglobulin (Ig) heavy chains and two Ig light chains. Several different types of heavy chain exist that define the class or isotype of an antibody. These heavy chain types vary between different animals. All heavy chains contain a series of immunoglobulin domains, usually with one variable (VH) domain that is important for binding antigen and several constant (CH) domains. Each light chain is composed of two tandem immunoglobulin domains: one constant (CL) domain and one variable domain (VL) that is important for antigen binding.
Antibodies can be described in terms of the CDRs of the heavy and light chains. As used herein, the term "CDR" or "complementarity determining region" is intended to mean the non-contiguous antigen combining sites found within the variable region of both heavy and light chain polypeptides. These particular regions have been described by Kabat et al., J. Biol. Chem. 252:6609-6616 (1977); Kabat et al., U.S. Dept. of Health and Human Services, "Sequences of proteins of immunological interest" (1991); by Chothia et al., J. Mol. Biol. 196:901-917 (1987); and MacCallum et al., J. Mol. Biol. 262:732-745 (1996), where the definitions include overlapping or subsets of amino acid residues when compared against each other. Nevertheless, application of either definition to refer to a CDR of an antibody or variants thereof, as described herein, is intended to be within the scope of the term as defined and used herein.

The term "isolated", when used as a modifier of an antibody of the invention means that the antibody is made by the hand of man or is separated, completely or at least in part, from their naturally occurring in vivo environment. Generally, isolated antibodies are substantially free of one or more materials with which they normally associate with in nature, for example, one or more protein. The term "isolated" does not exclude alternative physical forms of the antibodies, such as multimers/oligomers, modifications (e g , phosphorylation, glycosylation, lipidation) or derivatized forms, or forms expressed in host cells produced by the hand of man

An "isolated" antibody can also be "substantially pure" or "purified" when free of most or all of the materials with which it typically associates with in nature. Thus, an isolated antibody that also is substantially pure or purified does not include polypeptides or polynucleotides present among millions of other sequences, such as antibodies of an antibody library or nucleic acids in a genomic or cDNA library.

An "antigen binding fragment" comprises a portion of a full-length antibody. Examples of antigen binding fragments include Fab, Fab', F(ab')₂, and Fv fragments; diabodies; minobodies; nanobodies; linear antibodies (Zapata et al. (1995) Protein Eng. 8(10):1057-1062); single-chain antibody molecules; and multispecific antibodies formed from antibody fragments. The antigen binding fragment of the invention is preferably an anti-PUM2 protein binding fragment that reduces the levels and/or activity of the RNA-binding protein PUM2 e.g. by inhibiting or impairing the binding of the PUM2 protein to the target RNA. Preferably, said target RNA is an mRNA containing at least one motif 5•-UGUAXAUA-3• known as a Pumilio-binding element (PBE).

The terms "nucleic acid", "polynucleotide," and "oligonucleotide" are used interchangeably and refer to any kind of deoxyribonucleotide (e.g. DNA, cDNA, ...) or ribonucleotide (e.g. RNA, miRNA, siRNA, piRNA, hnRNA, snRNA, esiRNA, shRNA, ...) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are chemically modified in the base, sugar and/or phosphate moieties.
Chemical modifications of said oligonucleotides or nucleotides can comprise modifications to the nucleobases, the backbone residues, and/or the internucleoside linkers of said oligonucleotides.
Modifications to one or more nucleobases of said oligonucleotides may comprise one or more alkylated purines and pyrimidines, acylated purines and pyrimidines, and other heterocycles. These classes of pyrimidines and purines are known in the art and include pseudoisocytosine, N4,N4-ethanocytosine, 8-hydroxy-N-6-methyladenine, 4-acetylcytosine, 5-(carboxyhydroxylmethyl)uracil, 5 fluorouracil, 5-bromouracil, 5-carboxymethylaminomethyl-2-thiouracil, 5-carboxy-methylaminomethyl uracil, dihydrouracil, inosine, N6-isopentyl-adenine, 1-methyladenine, 1-methylpseudouracil, 1-methylguanine, 2,2-dimethylguanine, 2-methyladenine, 2-methylguanine, 3-methylcytosine, 5-methylcytosine, N6-methyladenine, 7-methylguanine, 5-methylaminomethyl uracil, 5-methoxy amino methyl-2-thiouracil, -D-mannosylqueosine, 5-methoxycarbonylmethyluracil, 5-methoxyuracil, 2 methylthio-N-6-isopentenyladenine, uracil-5-oxyacetic acid methyl ester, psueouracil, 2-thiocytosine, 5-methyl-2 thiouracil, 2-thiouracil, 4-thiouracil, 5-methyluracil, N-uracil-5-oxyacetic acid methylester, uracil 5-oxyacetic acid, queosine, 2-thiocytosine, 5-propyluracil, 5-propylcytosine, 5-ethyluracil, 5-ethylcytosine, 5-butyluracil, 5-pentyluracil, 5-pentylcytosine, and 2,6,diaminopurine, methylpsuedouracil, 1-methylguanine and 1-methylcytosine.
Modifications to one or more backbone residues of said oligonucleotides may comprise one or more of the following: 2' sugar modifications such as 2'-O-methyl (2'-OMe), 2'-O-methoxyethyl (2'-MOE), 2'-O-methoxyethoxy, 2'-Fluoro (2'-F), 2'-Allyl, 2'-O-[2-(methylamino)-2-oxoethyl], 2'-O-(N-methylcarbamate); 4' sugar modifications including 4'-thio, 4'-CH2-O-2'-bridge, 4-(CH2)2-O-2'-bridge; Locked Nucleic Acid (LNA); Peptide Nucleic Acid (PNA); Intercalating nucleic acid (INA); Twisted intercalating nucleic acid (TINA); Hexitol nucleic acids (HNA); arabinonucleic acid (ANA); cyclohexane nucleic acids (CNA); cyclohexenylnucleic acid (CeNA); threosyl nucleic acid (TNA); Morpholino oligonucleotides; Gap-mers; Mix-mers; Incorporation Arginine-rich peptides; addition of 5'-phosphate to synthetic RNAs; RNA Aptamers; or any combinations thereof.
Modifications to one or more internucleoside linkers of said oligonucleotides may comprise one or more of the following: Phosphorothioate, phosphoramidate, phosphorodiamidate, phosphorodithioate, phosphoroselenoate, phosphorodiselenoate, phosphoroanilothioate and phosphoranilidate, or any combinations thereof.
In general, an analogue of a particular nucleotide has the same base-pairing specificity; i.e., an analogue of A will base-pair with T. For example, a nucleic acid according to the invention, can be modified to enhance expression and reduce possible toxicity by including one or more modified nucleoside e.g. using pseudo-U or 5-Methyl-C.

In case said agent is a nucleic acid, then it will be selected among the non-limiting group comprising a miRNA, a siRNA, a piRNA, a hnRNA, a snRNA, a esiRNA, a shRNA, and an antisense oligonucleotide, or a combination thereof.

The terms "microRNA," "miRNA," and MiR" are interchangeable and refer to endogenous or artificial non-coding RNAs that are capable of regulating gene expression. It is believed that miRNAs function via RNA interference. The terms "siRNA" and "short interfering RNA" are interchangeable and refer to single-stranded or double-stranded RNA molecules that are capable of inducing RNA interference. SiRNA molecules typically have a duplex region that is between 18 and 30 base pairs in length.

The terms "piRNA" and "Piwi-interacting RNA" are interchangeable and refer to a class of small RNAs involved in gene silencing. PiRNA molecules typically are between 26 and 31 nucleotides in length.

The terms "snRNA" and "small nuclear RNA" are interchangeable and refer to a class of small RNAs involved in a variety of processes including RNA splicing and regulation of transcription factors. The subclass of small nucleolar RNAs (snoRNAs) is also included. The term is also intended to include artificial snRNAs, such as antisense derivatives of snRNAs comprising antisense sequences.

As used herein, an "shRNA molecule" includes a conventional stem-loop shRNA, which forms a precursor miRNA (pre-miRNA). "shRNA" also includes micro-RNA embedded shRNAs (miRNA-based shRNAs), wherein the guide strand and the passenger strand of the miRNA duplex are incorporated into an existing (or natural) miRNA or into a modified or synthetic (designed) miRNA. When transcribed, a conventional shRNA forms a primary miRNA (pri-miRNA) or a structure very similar to a natural pri-miRNA. The pri-miRNA is subsequently processed by Drosha and its cofactors into pre-miRNA. Therefore, the term "shRNA" includes pri-miRNA (shRNA-mir) molecules and pre-miRNA molecules.

siRNA and shRNA will preferably be selected among the non-limiting group of Table 1.

**Table 1**

| shRNA name | Sequence | Species | clone ID | SEQ ID No. |
|---|---|---|---|---|
| *PUM2*sh #1 | TTGACTTTATTCATCCATTT | Human | TRCN0000296986 | 1 |
| *PUM2*sh #2 | CTAGCTCCAACTGCCTATTAT | Human | TRCN0000297020 | 2 |
| *Pum2*sh #1 | CCTAATCCGACAGCGAATAAA | Mouse | TRCN0000233377 | 3 |
| *Pum2*sh #2 | ATTTGTGCCAAATCCATATAT | Mouse | TRCN0000233378 | 4 |
| scr | CAACAAGATGAAGAGCACCAA | Non-mammalian | SHC002 | 5 |
| | | | | |

| siRNA name | Sequence | Species | | |
|---|---|---|---|---|
| *siPUM2i #1* | AGAAAUAUGCCUUUUUGUCCA GACAAAAAGGCAUAUUUCUUG | Human | | 6 |
| *siPUM2 #2* | ACUGUUUCCAUGAAAACCCUG GGGUUUUCAUGGAAACAGUGA | Human | | 7 |
| *siPUM2 #3* | UUACUUCACUGUUUCCAUGAA CAUGGAAACAGUGAAGUAAAU | Human | | 8 |

As used herein, "antisense oligonucleotide" or "antisense sequence" refers to a single-stranded nucleic acid molecule having a nucleobase sequence that permits hybridization to a corresponding segment of a target nucleic acid. The antisense oligonucleotides are directed either against RNA (sense strand) or against DNA where they form triplex structures inhibiting transcription by RNA polymerase II.
Alternatively, the "antisense oligonucleotide" or "antisense sequence" can be a double-stranded antisense oligonucleotide and is also intended to be within the scope of the term as defined and used herein.
Any "antisense oligonucleotide" or "antisense sequence" known in the art, such as e.g. Gapmer (Lundin et al., in Hum Gene Ther. 2015 Aug 1; 26(8): 475-485) is also intended to be within the scope of the term as defined and used herein.

In a particular aspect of the invention, the nucleic acid is encoding at least one nuclease such as a Cas9 endonuclease, and at least one single guide RNA (sgRNA), or crRNA and tracrRNA, recognizing a DNA target sequence comprising 16 to 25 nucleotides wherein said DNA target sequence is selected from the group comprising the *Pum2* gene and a regulatory sequence of the Pum2 gene.
The Cas9 endonuclease of the present invention can be a naturally occurring Cas9, or variants and orthologs. Preferably, the Cas9 endonuclease of the present invention is a naturally occurring Cas9 endonuclease of S. pyogenes. In another aspect, the Cas9 endonuclease of the present invention comprises one or more mutations as compared to a naturally occurring Cas9. For example, the Cas9 can be a modified Cas9 endonuclease modified for transcriptional repression such as, e.g. an enzymatically dead Cas9. Specifically, both RuvC- and HNH- nuclease domains can be rendered inactive by point mutations (e.g. D10A and H840A in SpCas9), resulting in a nuclease dead Cas9 molecule that cannot cleave target DNA. However, the dead Cas9 molecule retains the ability to bind to target DNA based on the sgRNA targeting sequence, which sgRNA sequence is comprised in CRISPR-based gain/loss-of-function system.

Any nuclease of the gene editing system known in the art, such as a meganuclease, zinc finger nuclease (ZFNs), transcription activator-like effector-based nuclease (TALEN), CPF1 is also intended to be within the scope of the term as defined and used herein.

In a particular aspect of the invention, the nucleic acid is encoding at least one TALEN protein, wherein the at least one TALEN protein is capable of inducing a site-specific double stranded DNA break in a DNA target sequence selected from the group comprising the *Pum2* gene and a regulatory sequence of the *Pum2* gene.

In case said agent is a gene delivery vector, then said gene delivery vector comprises
i) a Cas9 endonuclease, and
ii) at least one single guide RNA (sgRNA), or crRNA and tracrRNA, recognizing a DNA target sequence comprising 16 to 25 nucleotides wherein said DNA target sequence is selected from the group comprising the *Pum2* gene and a regulatory sequence of the *Pum2* gene.
Preferably, the at least one sgRNA will be selected among the non-limiting group of sequences described in Table 2 or a variant or fragment thereof. Most preferably, the at least one sgRNA sequence is selected among the non-limiting group of Table 2.

**Table 2**

| sgRNA name | Sequence | Species | SEQ ID No. |
|---|---|---|---|
| *Pum2* sgRNA (#1) Fw | CACCGAGGCCTTGGTGTGAGCATGG | Human/Mouse | 9 |
| *Pum2 s*gRNA (#1) Rev | AAACCCATGCTCACACCAAGGCCTC | Human/Mouse | 10 |
| *Pum2* sgRNA #2 Fw | CACCGAGTTCCTCTCCTGCTGATAAA | Human/Mouse | 11 |
| *Pum2* sgRNA #2 Rev | AAACTTTATCAGCAGGAGAGGAACTC | Human/Mouse | 12 |
| *Pum2* sgRNA #3 Fw | CACCGGACAAAAAGGCATATTTCTT | Human/Mouse | 13 |
| *Pum2* sgRNA #3 Rev | AAACAAGAAATATGCCTTTTTGTCC | Human/Mouse | 14 |

Alternatively, the gene delivery vector comprises:
i) a nucleic acid encoding at least one TALEN protein, wherein the at least one TALEN protein is capable of inducing a site-specific double stranded DNA break in a DNA target sequence selected from the group comprising the Pum2 gene and a regulatory sequence of the Pum2 gene, and
ii) a nucleic acid donor sequence, wherein the donor sequence is selected among the non-limiting group of Table 3.

**Table 3**

| Sequence name | Sequence | Species | SEQ ID No. |
|---|---|---|---|
| *Pum2* TALEN (#1) | TGAGCTGCAGTTAAC spacer | Human | 15 |
| | TCAGAGGACTAATGCACTAA | | |
| *Pum2* TALEN (#2) | TGCAGTTAACTGTTG spacer | Human | 16 |
| | TCAGAGGACTAATGC | | |
| *Pum2* TALEN (#3) | TGCTGCTGTTGAGCT spacer | Human | 17 |
| | TGCACTAACAGTTCAAC | | |
| *Pum2* TALEN (#4) | TATGTGGCTGCTGAG spacer | Human | 18 |
| | TCAACAGCAGCAATATGCA | | |
| *Pum2* TALEN (#5) | TCAAAAGCACTCCCAAGA spacer | Human | 19 |
| | TAGGTTTACTCTGATT | | |
| *Pum2* TALEN (#6) | TGAGAACGTATGAAGA spacer | Human | 20 |
| | TAGCAGCAGCTCAGCAGCCA | | |

TALEN sequences were designed using the E-TALEN software and are compatible to be used with the Voytas Lab Golden Gate TALEN kit.
As described herein, the gene delivery vector of the invention is preferably selected from the non-limiting group comprising a viral and a plasmid vector.

As used herein, a "chemical agent" is a compound that produces change by virtue of its chemical composition and its effects on living tissues and organisms. The chemical agent may be a small molecule inhibitor (SMI) and is preferably a non-peptidyl molecule. Most preferably, the chemical agent is a small molecule inhibitor selected from the group comprising a tetracycline, actinonin, a phenicol, Urolithin A (UA), Carbonyl cyanide-4-(trifluoromethoxy)phenylhydrazone (FCCP) and a combination thereof.
Examples of tetracyclins include, but are not limited to, doxycycline, tetracycline, rolitetracycline, methacycline, minocycline and lymecycline. Typically, an effective amount of doxycycline to be administered systemically is about 50 mg/kg to about 100 mg/kg per day in the food for 9-10 weeks, preferably about 90 mg/kg/day in the food for 9-10 weeks. If doxycycline is administered in a liquid (e.g. water), then is will usually be administered in an effective amount of about 200 and 500 mg/kg/day in the water for 9-10 weeks.
Examples of phenicol include, but are not limited to, those selected from the group comprising chloramphenicol, thiamphenicol, florfenicol and a combination thereof.

Also contemplated in the present invention is a pharmaceutical composition comprising a therapeutically effective amount of at least one agent of the invention that reduces the levels and/or activity of the RNA-binding protein PUM2. Preferably, the agent that reduces the levels and/or activity of the RNA-binding protein PUM2 of the invention is selected from the group comprising a chemical agent, a peptide or analog thereof, an antibody or an antigen-binding fragment of said antibody, a nucleic acid, a gene delivery vector, and a combination thereof.
Preferably, the pharmaceutical composition is intended for use in treating or preventing an aging-related disease described herein.

The term "pharmaceutical" as used herein refers to a chemical substance intended for use in the cure, treatment, or prevention of disease. Details on techniques for formulation and administration of such compositions may be found in Remington, The Science and Practice of Pharmacy 21st Edition (Mack Publishing Co., Easton, Pa.) and Nielloud and Marti-Mestres, Pharmaceutical Emulsions and Suspensions: 2nd Edition (Marcel Dekker, Inc, New York).
For the purposes of this disclosure, the pharmaceutical compositions may be administered by a variety of means including orally, parenterally, by inhalation spray, topically, or rectally in formulations containing pharmaceutically acceptable carriers, adjuvants and vehicles. The term parenteral as used here includes but is not limited to subcutaneous, intravenous, intramuscular, intraarterial, intradermal, intrathecal and epidural injections with a variety of infusion techniques. Intraarterial and intravenous injection as used herein includes administration through catheters. Administration via intracoronary stents and intracoronary reservoirs is also contemplated. The term oral as used herein includes, but is not limited to oral ingestion, or delivery by a sublingual or buccal route. Oral administration includes fluid drinks, foods, energy bars, as well as pill formulations
Pharmaceutical compositions may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing a drug compound in admixture with non-toxic pharmaceutically acceptable excipient which are suitable for manufacture of tablets are acceptable. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents; such as magnesium stearate, stearic acid or talc. Tablets may be uncoated, or may be coated by known techniques including enteric coating, colonic coating, or microencapsulation to delay disintegration and adsorption in the gastrointestinal tract and/or provide a sustained action over a longer period. For example, a time delay material such as glyceryl monostearate or glyceryl distearate alone or with a wax may be employed.
Formulations for oral use may be also presented as hard gelatin capsules where the drug compound is mixed with an inert solid diluent, for example calcium phosphate or kaolin, or as soft gelatin capsules wherein the active ingredient is mixed with water or an oil medium, such as peanut oil, liquid paraffin or olive oil.
Such excipients include a suspending agent, such as sodium carboxymethylcellulose, methylcellulose, hydroxypropyl methylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.
Oil suspensions may be formulated by suspending the active ingredient in a vegetable oil, such as arachis oil, olive oil, sesame oil or coconut oil, or a mineral oil such as liquid paraffin. The oral suspensions may contain a thickening agent, such as beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.
Dispersible powders and granules of the disclosure suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.
The pharmaceutical compositions of the disclosure may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion may also contain sweetening and flavoring agents.
Syrups and elixirs may be formulated with sweetening agents, such as glycerol, sorbitol or sucrose. Such formulations may also contain a demulcent, a preservative, a flavoring or a coloring agent.
The pharmaceutical compositions of the disclosure may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a non-toxic parenterally acceptable diluent or solvent such as a solution in 1,3-butane-diol or prepared as a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.
The amount of active ingredient, i.e. the agent that reduces the levels and/or activity of the RNA-binding protein PUM2 as described herein, which may be combined with the carrier material to produce a single dosage form will vary depending upon the host treated and the particular mode of administration. For example, a time-release formulation intended for oral administration to humans may contain approximately 20 to 500 mg of active material compounded with an appropriate and convenient amount of carrier material which may vary from about 5 to about 95% of the total compositions. It is preferred that the pharmaceutical composition be prepared which provides easily measurable amounts for administration. Typically, an effective amount to be administered systemically is about 0.1 mg/kg to about 100 mg/kg and depends upon a number of factors including, for example, the age and weight of the subject (e.g., a mammal such as a human), the precise condition requiring treatment and its severity, the route of administration, and will ultimately be at the discretion of the attendant physician or veterinarian. It will be understood, however, that the specific dose level for any particular patient will depend on a variety of factors including the activity of the specific compound employed, the age, body weight, general health, sex and diet of the individual being treated; the time and route of administration; the rate of excretion; other drugs which have previously been administered; and the severity of the particular condition undergoing therapy, as is well understood by those skilled in the art.

The term "therapeutically effective amount" as used herein means an amount of an agent that reduces the levels and/or activity of the RNA-binding protein PUM2 high enough to significantly positively modify the symptoms and/or condition to be treated, but low enough to avoid serious side effects (at a reasonable risk/benefit ratio), within the scope of sound medical judgment. The therapeutically effective amount of agent of the invention is selected in accordance with a variety of factors including type, species, age, weight, sex and medical condition of the patient; the severity of the condition to be treated; the route of administration; the renal and hepatic function of the patient. A physician of ordinary skill in the art can readily determine and prescribe the effective amount of the agent required to prevent, counter or arrest the progress of the concerned disease.

Alternatively, the pharmaceutical composition of the invention may comprise a therapeutically effective amount of a cell or a population of cells. Preferably, said cell or population of cells comprise a modification or alteration of a DNA target sequence comprising 16 to 25 nucleotides of interest, wherein said DNA target sequence is selected from the group comprising the *PUM2* gene and a regulatory sequence of the *PUM2* gene.

Alternatively also, the pharmaceutical composition of the invention may comprise a therapeutically effective amount of a cell or a population of cells, wherein said cell or population of cells comprises a gene delivery vector of the invention. Preferably, the gene delivery vector is selected from the non-limiting group comprising a viral and a plasmid vector as described herein.
The pharmaceutical compositions comprising a therapeutically effective amount of a cell or a population of cells are administered to the patient by any route of administration and/or delivery methods known in the art and/or described herein.

The present invention further contemplates a method of treating or preventing an aging-related disease comprising modifying the DNA target sequence disclosed herein in a single cell or a population of cells, and reintroducing the modified single cell or population of cells into the patient in need thereof.
Preferably, a biopsy or other tissue or biological fluid sample comprising the single cell or the population of cells may be necessary. Stem cells such as ES cells or pluripotent stem cell that can be generated directly from adult cells, such as iPSCs, are particularly preferred in this regard. Usually, the cell is selected - or derived - from the group comprising neurons, glia, satellite muscle cells, heart cells, hepatocytes, and fibroblasts.
The modified single cell or population of cells is/are then reintroduced into the patient in need thereof by any route of administration and/or delivery methods known in the art as described below.

In yet another aspect, the invention provides kits for treating or preventing an aging-related disease as described herein, said kits comprising
i) a composition or a pharmaceutical composition of the invention,
ii) a gene delivery vector of the invention, or
iii) an acid nucleic of the invention,
iv) or a combination thereof.
The kits of the invention may also comprise a container and a label or package insert on or associated with the container. Suitable containers include, for example, bottles, vials, syringes, etc. The containers may be formed from a variety of materials such as glass or plastic. The container holds a composition which is effective for treating the disease of disorder of the invention and may have a sterile access port (for example the container may be an intravenous solution bag or a vial having a stopper pierceable by a hypodermic injection needle). Alternatively, or additionally, the kits may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The label or package insert may comprise instructions for use thereof. Instructions included may be affixed to packaging material or may be included as a package insert. While the instructions are typically written or printed materials they are not limited to such. Any medium capable of storing such instructions and communicating them to an end user is contemplated by this disclosure.

The present invention further contemplates the use of a pharmaceutical composition of the invention in the manufacture of a medicament. Preferably, the medicament is for treating or preventing an aging-related disease.

Since the present invention can be effective in the cosmetic as well, further comprised in the present invention is a cosmetic composition comprising a cosmetically effective amount of at least one agent of the invention that reduces the levels and/or activity of the RNA-binding protein PUM2. Preferably, the agent that reduces the levels and/or activity of the RNA-binding protein PUM2 of the invention is selected from the group comprising a chemical agent, a peptide or analog thereof, an antibody or an antigen-binding fragment of said antibody, a nucleic acid, a gene delivery vector, and a combination thereof as described herein.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein. Various references are cited throughout this Specification, each of which is incorporated herein by reference in its entirety. The foregoing description will be more fully understood with reference to the following Examples.

### EXAMPLES

### Experimental Model and Subject Details

Mice: 24 months old male C57BL/6J mice (Janvier) were bilaterally injected in the gastrocnemius muscles with 5*10¹¹ viral particles containing the g*Pum2* #1 (left leg) or the px601 vector as control (right leg) (n = 7). Mice were housed single-caged. After 5 weeks, animals were randomized, sacrificed and processed to perform electron microscopy imaging (n=2 per condition) and for all biochemical and functional analyses described below (n=5 per condition). For the latter analyses, gastrocnemius muscles were rapidly removed and snap-frozen in liquid nitrogen. 2 months and 24 months old male C57BL/6J mice (Janvier) (n=3) were sacrificed and whole brain and tibilialis anterioris tissues were OCT-embedded for immunohistochemical analysis, while quadriceps and forelimb muscles were rapidly snap-frozen. No blinding was used during the experiment procedures. All experiments were performed in compliance with all relevant ethical regulations. The mice experiments were authorized by the local animal experimentation committee of the Canton de Vaud, under license 2890. The committee that approved the license is the "Commission cantonale pour l'expérimentation animale".

Cell cultures: C2C12 myoblasts (female, ECACC, 91031101) were cultured in Dulbecco's modified Eagle's medium with high glucose content (Gibco, DMEM, 41966-029), 20% of Fetal Calf Serum (FCS) and 100U/mL penicillin and 100µg/mL streptomycin (Gibco, 15140-122). HeLa (female, ATCC® CCL-2™), HEK-293T (ATCC® CRL-3216™) and HEK AAV-293 cells (Agilent, 240073) cells were cultured in the same condition described above, but with 10% FCS. HeLa and C2C12 stable clones were cultured as described above with the addition of puromycin 2 • g/ml (Invivogen, QLL-38-03A) as a selection antibiotic. Trypsin-EDTA 0.05% (Gibco, 25300-062) was used for all the cell lines. All cells were maintained at 37°C and 5% CO₂.

*C. elegans* Strains and Bacterial Strains: *C. elegans* strains were provided by the Caenorhabditis Genetics Center (University of Minnesota). N2 Bristol, SJ4103 (zcIs14[myo-3::GFP(mit)]) and RW1596 (stEx30 [myo-3p::GFP::myo-3 + rol-6(su1006)]) strains were cultured at 20 °C. The *glp-1*(e2144) strain was cultured at 15°C. All strains were cultured on nematode growth media agar plates seeded with *E. coli* strain OP50. To perform the experiments, *glp*-*1*(e2141ts) eggs were seeded on plates and raised at 25°C (to eliminate germ cells) for • 40 hours, then shifted to 20°C for the rest of life. Bacterial RNAi feeding experiments were carried out as described in (Kamath et al., 2001), from Day 1 of adulthood, unless differently specified. Clones used were puf-8 (C30G12.7) and mff-1 (F11C1.2). Clones were purchased from Source BioScience and verified by sequencing.

### Method details

*C. elegans* motility, survival and oxygen-consumption assays: *C*. *elegans* movement analysis was performed as described in (Mouchiroud et al., 2016), starting from day 1 of adulthood, using the Movement Tracker software. The experiments were repeated at least twice. Lifespan tests were performed at 20 °C as described in (Mouchiroud et al., 2013). Worms that crawled off the plate or had an exploded vulva phenotype were censored. These exclusion criteria were established before starting the experiments. Oxygen consumption was measured using the Seahorse XF96 respirometer (Seahorse Bioscience) (Koopman et al., 2016). Maximal respiration was measured after injecting carbonyl cyanide-4(trifluormethoxy)phenylhydrazone (FCCP, C2920, Sigma-Aldrich) at a final concentration of 10 • M in the well. Respiration rates were normalized to the number of worms in each individual well and calculated as averaged values of 6-7 repeated measurements. Each experiment was repeated at least twice.

Cell transfection and transduction: Lentiviruses were produced by cotransfecting HEK293T cells with lentiviral, packaging (pCMV-dR8.2 dvpr, Addgene, #8455) and envelope (pCMV-VSV-G, Addgene, #8454) plasmids, in a ratio of 4:3:1, respectively, by using FuGENE 6 (Promega, E2691) following manufacturer's instructions. Transfection medium was removed 24 h after transfection and fresh medium was added to the plate. Cell supernatants were collected at 24 and 48 h and filtered through a 0.45-• m filter. Cells to be transduced were seeded 24 hours before infection and then transduced with virus-containing supernatant supplemented with 5 mg/ml of polybrene (Millipore). This step was repeated twice and cells were left to recover for 24 h in growth media before puromycin (2 • g/ml) selection. The list of shRNAs are shown in Supplementary Table 4. HEK293T and were transfected with the indicated amounts of EGFP-PUM2 or pEGFP-C1, as control, for 24 hours, using FuGENE6. To assess mitophagy, HEK293T were transfected with m-KEIMA (pCHAC-mt-mKeima, Addgene #72342) together with the pCL-Ampho Retrovirus Packaging Vector (Novus Biologicals) in a ratio 1:1, by using FuGENE 6. Cell supernatants were collected at 48 h and filtered through a 0.45-• m sterile filter. C2C12 cells were transduced with the virus-containing supernatant, left to recover for 24 h in growth media and analyzed by confocal imaging. For siRNA transfection in C2C12, Lipofectamine® RNAiMAX (Thermo Scientific, 13778100) was used following manufacturer's instructions. 50 picomoles of the siRNA listed in Supplementary Table 4 (synthetized by Microsynth, custom made) were transfected and cells were analyzed by confocal imaging after 48 hours.

Western blot analysis: Cells and tissues were lysed in RIPA buffer [50 mM tris (pH 7.4), 150 mM NaCl, 5 mM EDTA, 0,1% SDS, 1% NP40, 100 mM NaF, 5mg/ml Sodium deoxycholate] with HaltTM protease (Thermo Fisher Scientific, 78430) and phosphatase (Thermo Fisher Scientific, 78442) inhibitor cocktails. Clear supernatants were collected and protein concentration was assessed by DC protein assay (Bio-Rad, 500-0116). Lysates were eluted in 5x Laemmli buffer (715um • -mercapto-ethanol, 1M Tris-HCl pH 6.8, 30% Glycerol, 10% SDS, 0.25% Blu Bromophenol). Equal amounts of proteins were separated by SDS-PAGE and transferred onto polyvinylidene difluoride membranes (Millipore, Immobilion-P IPIVH00010). Filters were washed in TBS + 0,05% Tween and blocked for 1 hour with 5% Non-Fat milk. For co-immunoprecipitation assays, cells and tissues were lysed in CoIP buffer (50mM Tris-HCl pH 7.4, 75mM NaCl, 1% NP-40, 0,1% SDS, 5mg/ml Sodium deoxycholate. 1ml of CoIP buffer for 20 million of cells, 5ml for one whole brain lysate) plus protease and phosphatase inhibitor cocktails. 1mg of clear supernatants from cells or 2mg from whole brain extracts were precleared with 30ul of Protein A/G Agarose beads (Pierce, 20421), 2ug of rabbit Igg (Santacruz, sc-2027) and 0,05% BSA and incubated 1 hour at 4°C on a rotating wheel. 2.5% of the precleared lysates were used as input. The rest of the precleared extracts were incubated with 5ug of either an antibody against PUM2 (Abcam, ab10361) or of rabbit control IgG (Santacruz, sc-2027) overnight at 4°C on a rotating wheel. Samples were added with 50ug of Protein A/G Agarose and incubated on the rotating wheel for additional 1 hour. Beads were washed 5 times in CoIP buffer, eluted in 5x Laemmli Buffer and boiled at 95°C. Supernatants were analyzed by western blot as described above. Proteins were detected using the following antibodies: PUM2 (Santacruz, sc-514108, 1:1000), MFF (ProteinTech Group, 17090-1-AP, 1:1000), OXPHOS cocktail (Mitoscience/Abcam, ab110413, 1:2000), PABPC1 (Abcam, ab21060, 1:3000), PINK1 (Novus Biologicals, BC100-494, 1:500), SQSTM1/p62 (Progen, GP62-C, 1:500), LC-3 (Cell Signaling, 4108S, 1:500), GAPDH (Cell Signaling, 2118,1:3000), HSP90 (BD Transduction Laboratories, 610418, 1:4000). The secondary antibodies used were goat-anti-rabbit (Cambridge Bioscience, AC2114, 1:5000), goat-anti-mouse (Cambridge Bioscience, AC2115, 1:5000), donkey-anti-goat (Cambridge Bioscience, AC2149, 1:2500) and goat-anti-Guinea pig H&L HRP (Abcam, ab6908, 1:2500) HRP secondary antibodies. Antibody reactions detection were imaged using the c300 imaging system (Azure Biosystems).

Tissue, worm and cell immunofluorescence analysis: Tissue immunostaining was performed on fresh frozen section of mice tibialis anterior muscles and whole brain tissues. 8um sections were cut from OCT-embedded samples. Sections were fixed with 4% PFA for 20 min, permeabilized in PBS + 0,5% Triton-X-100 and blocked with 3% BSA+ 10% donkey serum for 1 hour at RT. Sections were stained using an antibody against PUM2 (Abcam, ab10361, 1:300) overnight at 4°C and incubated with the secondary antibody (donkey anti-rabbit Alexa 568, 1:1000) 1 hour at RT. A final counterstaining with Hoechst (Thermo Scientific, 33342, 1:10000) was performed for 3 min. ThS staining was performed for 15 minute RT (ThS 0,03% in 50% EtOH), prior primary antibody incubation. p_{myo-3}::mito::GFP worms at the indicated ages were prepared for imaging as described in (Houtkooper et al., 2013). C2C12 clones and HEK293T were fixed with 4% PFA for 20 minutes, permeabilized with 0.1% Triton-X-100 and blocked with 3% BSA 1 hour at RT. For mitochondrial morphology analysis, cells were stained with Tom20 antibody (Santa Cruz, sc-11415, 1:200) overnight at 4°C, incubated with the secondary antibody (anti-rabbit Alexa Fluor-488, 1:1000) for 1 hour at RT. Images were obtained analyzing z-stacks (max intensity) at multiple positions under non-saturating exposure conditions. For stress granules imaging, C2C12 were hybridized with a probe targeting the mouse *Mff* mRNA (ACD, RNAscope® Probe- Mm-Mff, Cat No. 510891), which was detected with the RNAscope® Fluorescent Multiplex kit following manufactures' instructions (ACD, Cat No. 320850). *Ppib* mRNA (ACD, Cat No. 313911) and *Dapb* mRNA (ACD, Cat No. 310043) were used as positive and negative controls, respectively. Cells were then stained for PUM2 (Abcam ab10361, 1:300). For imaging of the dose-dependent accumulation of SGs, HEK293T were transfected with different amounts of GFP proteins and stained with a PABPC1 antibody (Abcam, ab21060, 1:200) overnight at 4°C, and incubated with the secondary antibody (donkey anti-rabbit Alexa 568, 1:1000) for 1 hour at RT. For all cell immunofluorescence staining, nuclei were counterstained with DAPI. To analyze stress granules number and area, GFP-PUM2 and PABPC1 co-stained samples were thresholded and double positive granules were counted for at least 20 cells from three random fields to assess the number of SGs per cell. Co-positive PUM2 and ThS (Sigma, T1892) granules in the neocortex, hippocampus and dentate gyrus regions of mice brain tissues were counted and the stress granules area was calculated by using the Fiji "analyse particles" for > 20 cells. Image processing was performed with Fiji.

Mitophagy quantitation: C2C12 transduced with the m-KEIMA were examined as described in (Zheng et al., 2017). In brief, m-Keima fluorescence was imaged in two channels via two sequential excitations (488 nm, cyan; 555 nm, red) and using a 570- to 695-nm emission range. Imaging settings were maintained with the same parameters for comparison between different experimental conditions. Mitophagy index quantification was performed as previously described (Sun et al., 2015). Briefly, 550/438 signal ratio was calculated for each region as 550/(550+438) signal, background corrected using a not m-KEIMA transfected control condition and normalized by total cell area. The average of 20-30 cells from each condition was taken, and the values were normalized to the average value seen in the control, assigned the value of one. In each experimental condition, imaging parameters are the same for all data acquisitions.

Luciferase assay : The full-length 3•UTR of human *MFF* mRNA was amplified by PCR with the following primers:
MFF_3'UTR_Fw: 5' TTTTCTAGAAGGTAACATCAGCCCTC 3' (SEQ ID No 21);
MFF_3'UTR_Rev: 5' TTTTCTAGATTCTTTGGTTGACAGTTTATTAAATAC 3' (SEQ ID No 22).
The PCR product was subcloned into the pGL3-promoter vector (Promega) using the XbaI restriction enzyme (NEB, R0145) and sequenced to check the correct directionality of the inserted sequence. For the luciferase assay, Hela SCR and PUM2sh stable clones were seeded in 96 well plates and transfected with 100ug of either the pGL3-promoter (Promega) or the pGL3-Luc-MFF 3'UTR plasmid. In all conditions, the cells were co-transfected with 10ug of pRL-CMV Renilla-expressing vector. FuGENE 6 (Promega) was used as transfection reagent, following manufacturer's instructions. Cells were analyzed for both Luciferase and Renilla luminescence using the Dual-Glo® Luciferase Assay System Protocol (Promega, E2940), according to the manufacturer's instructions.

Quantitative real-time PCR (q-RT-QPCR), mtDNA/nDNA ratio and citrate synthase activity. RNA from cells and tissues and worms was extracted using TRIzol (Thermo Scientific, 15596026) and then transcribes in cDNA by the QuantiTect Reverse Transcription Kit (Qiagen, 205313) following the manufacturer's instructions. The q-RT-PCR reactions were performed using the Light-Cycler system (Roche Applied Science) and the expression of selected genes was analysed using the LightCycler 480 System (Roche) and SYBR Green chemistry. All quantitative polymerase chain reaction (PCR) results were presented relative to the mean of housekeeping genes (• • Ct method). mRNA levels were normalized over *Gapdh* for gene expression for cell and tissue samples. For *C*. *elegans,* two housekeeping genes were used to normalize the expression data, actin (*act-1*) and peroxisomal membrane protein 3 (*pmp-3*). Relative values for mitochondrial genes (*mt-16S*) and nuclear genes (*Hk2*) were compared in each sample to generate a ratio representing the relative level of mtDNA per nuclear genome. The average of three technical replicates was used for each biological data point. Primer sets for quantitative reverse transcription PCR (q-RT-PCR) analyses are shown in Supplementary Table 4. Citrate synthase (CS) activity was determined using the CS assay kit (Sigma, CS0720-1KT). Absorbance at 412•nm was recorded on a Victor X4 (PerkinElmer) with 10 readings over the 1.5-min timespan. These readings were in the linear range of enzymatic activity. The difference between baseline and oxaloacetate-treated samples was obtained and used to calculate total citrate synthase activity according to the formula provided in the manual. The obtained values were normalized to the amount of protein used for each sample.

RNA-Crosslinking and Immunoprecipitation (CLIP): C2C12 cells were plated on 150 cm culture plate and after 24 hours the medium was replaced by ice cold PBS. For CLIP from brain tissues, one brain per experimental condition was resuspended in ice-cold PBS and chunked in smaller pieces. The solution was pipetted with a glass Pasteur pipette until dissolving all visible tissue pieces and the layered on a 10 cm culture plate. For both C2C12 cells and tissue homogenate, plates were exposed to 150 mJ/cm2 UVC (Stratagene, model UV Stratalinker 2400) on ice. Cells were then pelleted and lysed in CLIP buffer (50 mM Tris-HCl pH 7.4, 100 mM NaCl, 1% NP-40, 0.1% SDS, 0.5% sodium deoxycholate, 80 U/ml RNase OUT (Invitrogen) with protease inhibitor). Supernatants were pre-cleared with Protein A/G beads, rabbit control IgG, 0.05% BSA (Sigma, A7906-100G) and 0.2 • g/ml yeast tRNA (Thermo Scientific, AM7119). Pre-cleared lysates were incubated with PUM2 antibody (Abcam, ab10361, 5 • g) or the corresponding amount of control rabbit IgG, overnight at 4°C with gentle rotation. Next day, 40 • l of Protein A-G beads were added to the lysates and the tubes were incubated for an additional hour at 4°C with gentle rotation. Then, beads were washed 5 times with CLIP buffer. Beads were treated using 20 units of RNase-free DNase (Roche, 04716728001) for 15 min at room temperature, followed by PCR-grade Proteinase K (50 • g, Roche, RPROTKSOL-RO) treatment for 30 min at 37° C. Immunoprecipated RNA was isolated using miRNeasy kit (Qiagen, 217004) and q-RT-PCR was performed using primers designed to amplify *Mff* cDNA regions flaking the PUM2-binding site. Primers are listed in Supplementary Table 4. Isolated RNA from a fraction (5%) of pre-cleared lysate was used as input.

SILAC proteome analysis: For SILAC labeling, HeLa SCR and *PUM2sh* #1 clones were cultured in for six passages in SILAC DMEM media minus 1-arginine and 1-lysine (Thermo Scientific, 89985) with 10% dialyzed fetal bovine serum (Invitrogen) and 100 U/mL penicillin/streptomycin. Two biological replicates were performed, namely Forward (Fw) and Reverse (Rev), with the cells containing the following mixture of amino acids at 49 mg/mL each: SCR Fw, 1-arginine (Arg0) and 1-lysine (LysO); *PUM2*sh Fw, 13C615N4-1-arginine (Arg10) and 13C615N2-1-Lysine (Lys8); SCR Rev, 13C615N4-1-arginine (Arg10) and 13C615N2-1-Lysine (Lys8), *PUM2*sh Rev, 13C614N4-1-arginine (Arg6) and 4,4,5,5-D4-1-lysine (Lys4). The SILAC-labeled cells were then lysed in SDS 2% and protein concentrations of the different lysates were estimated with DC protein assay. Samples were mixed equitably at 10• g per SILAC state per replicate. Samples were trypsin digested using the FASP protocol(Wi•niewski et al., 2009). Peptide were desalted on C18 StageTips and fractionated into six fractions by strong cation exchange. All fractions were dried down by vacuum centrifugation prior to Mass Spectrometric measurements. For LC MS/MS analysis, peptides were resuspended in 2% acetonitrile, 0.1% FA and separated by reversed-phase chromatography on a Dionex Ultimate 3000 RSLC nanoUPLC system in-line connected with an Orbitrap Q Exactive Mass-Spectrometer (Thermo Fischer Scientific). SILAC labeled data were analyzed with MaxQuant version 1.5.1.2 against a concatenated database consisting of UniProt human database release 2015_07. Carbamidomethylation was set as a fixed modification, whereas oxidation (M) and acetylation (Protein N-term) were considered as variable modifications. Relative quantification within different conditions was obtained calculating the significance B values for each of the identified proteins using Perseus software (Tyanova et al., 2016). Supplementary Table 1 contains the full proteomic dataset.
HeLa stable clones were seeded at density of 3 x 10⁵ cells/well in 6-well plates. After 48 hours, cells were washed twice with 75 mM ammonium carbonate buffer (pH 7.4, 37°C) and cell metabolism was quenched by shock freezing of plates in liquid N₂. Metabolites were extracted by adding 700 ul ice-cold acetonitrile/methanol/water (40:40:20, v/v). The extraction procedure was repeated twice. Scraped cells and supernatants were pooled in the same tube. To remove cell debris, tubes were centrifuged (4°C, 13'000 rpm, 2 min) and the supernatants collected were assayed by flow injection analysis using TOF MS (6550 QTOF, Agilent Technologies) operated in the negative ionization mode. High resolution mass spectra were recorded from 50-1,000 m/z and analyzed as described previously (Fuhrer et al., 2011). Detected ions were putatively annotated by accurate mass against the Human Metabolome Database version 3.6 (Wishart et al., 2013). Mass matching was done using a tolerance of m/z 0.001 and including isotopologues, common electrospray derivatives, and adducts. Differences between treatments and control cells were determined using the R/Bioconductor package limma.

RNA sequencing library preparation and analysis: Total RNA from HeLa cells was extracted using TRIzol (Thermo Fisher Scientific) and purified by column using RNeasy Mini kit (QIAGEN, 74104). Agilent 2100 Bioanalyzer (Agilent RNA 6000 Nano Kit) was used to detect the total RNA samples concentration, RIN, 28S/18S and size. The purity of the samples was tested by NanoDrop™. Total RNA sample was digested by DNase• (NEB) and purified by oligo-dT beads (Dynabeads mRNA purification kit, Invitrogen, 61006), then poly (A)-containing mRNA were fragmented into 130bp with First Strand buffer. First-strand cDNA was generated by N6 primer, FirstStrand Master Mix and Super Script II reverse transcription (Invitrogen, 18064022) (Reaction condition• 25°C for 10 min• 42°C or 40 min• 70°C for 15 min). Then Second Strand Master Mix was added to synthesize the second-strand cDNA (16°C for 1h). The cDNA was purified with Ampure XP Beads (Agencourt, A63880), combined with End Repair Mix, incubated at 20°C for 30min, purified, added with A-Tailing Mix and incubated at 37°C for 30 min. Then the samples were combined with Adenylate 3' Ends DNA, Adapter and Ligation Mix and incubated for the ligation reaction at 20°C for 20 min. Several rounds of PCR amplification with PCR Primer Cocktail and PCR Master Mix were performed to enrich the cDNA fragments. Then the PCR products were purified with Ampure XP Beads. The final library was quantitated in two ways: determining the average molecule length using the Agilent 2100 bioanalyzer (Agilent DNA 1000 Reagents), and quantifying the library by real-time quantitative PCR (QPCR) (TaqMan Probe). The libraries were amplified on cBot to generate the cluster on the flow cell (TruSeq PE Cluster Kit V3-cBot-HS, Illumina, PE-401-3001) and the amplified flow cell was sequenced pair end on the HiSeq 2000 System (TruSeq SBS KIT-HS V3 π Illumina, TG-101-3001), with read length 50bp. The reads obtained were first trimmed out to remove adaptor sequences and then mapped using a TopHat2 against the human genome (GRCh37). HTSeq-count was then used to obtain the counts of each gene. Differential expressed genes in each condition were identified using R/Bioconductor package Deseq.

Optiprep Density Gradient Centrifugation: Whole brains were lysed using extraction buffer (EB, 25 mM HEPES [pH 7.3], 150 mM KCl, 8% glycerol, 0.1% NP-40, 1 mM DTT, protease inhibitor cocktail [Roche], 40 U/ml RNase inhibitors), using a motor-driven dounce as described in (Fritzsche et al., 2013). Soluble supernatant of homogenized brain lysate was layered on top of a 10 ml 15%-30% linear Optiprep (Axis Shield, 1114542) density gradient and centrifuged in a swinging bucket rotor (SW41, Beckman Coulter Genomics) at 280,000 × g at 4°C for 2.5 hr. Eight fractions of 900•1 each were collected from the top to the bottom of the gradient and flash-frozen in liquid nitrogen. Samples of each fraction were boiled in Laemmli Buffer and subjected to western blotting.

CRISPR-Cas9 plasmid cloning and AAV production: Guide RNAs targeting the *Pum2* gene were designed using the CRISPR Benchling design software (https://benchling.com/crispr, gRNA length = 21bp with additional 5•guanine, PAM = NNGRRT), selecting the first 3 hits, ranked by the On-target score. The primers were designed and cloned in the HA-saCas9/containing px601 plasmid, as described in (Ran et al., 2015), to obtain the following plasmids: g*PUM2* #1, #2 and #3. The primers used for the cloning are listed in the Supplementary Table 4. For the guide efficiency determination, 3ug of g*PUM2*sh#1, #2, #3 and px601 as control were transfected in HEK293T with Fugene6, following manufacturer's instructions. After 48 hours, cells were lysed in RIPA buffer and analyzed by Western Blot as described above. g*PUM2* #1 was selected as the most efficient guide and its genome editing efficiency was further confirmed by sequencing the genomic region containing the g*PUM2* #1-targeted sequence. Genomic DNA from cells transfected with the g*PUM2*#1 and the px601 plasmids was extracted with the NucleoSpin® Tissue kit (Macherey-Nagel, 740952). The PCR was performed using the oligos listed in the Supplementary Table 4. The PCR product was gel purified and Sanger sequenced with the PCR Fw oligo. PCR products were analyzed for % of indels and of aberrant sequences using TIDE (https://www.deskgen.com/landing/tide.html). Recombinant serotype 9 adeno-associated viral (AAV) vectors were produced according to standard procedures, as described in (Löw et al., 2013). Briefly, HEK AAV-293 cells (Agilent) were co-transfected with the pAAV (px601 or px601-g*PUM2*sh#1) and pDP9 plasmids (Sonntag et al., 2011). The AAV9 particles contained in the cell lysates were isolated on an iodixanol gradient followed by ion exchange FPLC using a HiTrap Q-FF column (5 ml, GE Healthcare) connected to an AKTA start chromatography system (GE Healthcare). After buffer exchange (resuspension in DPBS) and concentration on a centrifugal filter (cut-off 100 kDa, Amicon Ultra, Millipore), the vector suspensions were titered by real-time PCR for the presence of genome-containing particles (VG), as described in (D'Costa et al., 2016). The titers of the obtained AAV9 suspensions used for in vivo experiments ranged between 7.9E13 VG/mL and 2.4E14 VG/mL.

Transmission electron microscopy: 24 months old male C57BL/6J mice were perfused, via the heart, with 200 ml of a buffered mix of 2.5 % glutaraldehyde and 2.0 % paraformaldehyde in 0.1M phosphate buffer (pH 7.4), and then left for 30 minutes. The gastrocnemius muscle was then removed and the medial portion sliced into small (1 x 1 x 1 mm) cubes which were then further fixed in the same fixative solution for a further 4 hours. The cubes were then washed thoroughly with cacodylate buffer (0.1M, pH 7.4), postfixed for 40 minutes in 1.0 % osmium tetroxide with 1.5% potassium ferrocyanide, and then in 1.0% osmium tetroxide for a further 40 minutes alone. They were finally stained for 30 minutes in 1% uranyl acetate in water before being dehydrated through increasing concentrations of alcohol and then embedded in Durcupan ACM (Fluka, Switzerland) resin. The samples were then placed in silicon molds and left to harden for 24 hours in a 65°C oven. Thin, 50 nm thick, sections were cut with a diamond knife, and collected onto pioloform support films on single slot copper grids. These were contrasted with lead citrate and uranyl acetate, and images taken with a transmission electron microscope at 80 kV (Tecnai Spirit, FEI Company with Eagle CCD camera). To analyse the morphology of mitochondria we used block face scanning electron microscopy. The fixed muscle tissue was cut into small cubes (1 mm x 1 mm) and immersed in a buffered mix of paraformaldehyde (1%) and glutaraldehyde (1.25%). After 24 hours, the pieces were postfixed in potassium ferrocyanide (1.5%) and osmium (2%), then stained with thiocarbohydrazide (1%) followed by osmium tetroxide (2%) and then stained overnight in uranyl acetate (1%). Sections where washed in water at 50°C before being stained with lead aspartate at the same temperature. The sections were then dehydrated in increasing concentrations of ethanol and then embedded in spurs resin which was hardened at 65 degrees for 24 h. Samples were glued to aluminum mounting pins and mounted inside the electron microscope (Zeiss Merlin) fitted with a 3View system (Gatan). Serial images were collected of the region of interest at 1.5 kV using a pixel size of around 6 nm and 50 nm of resin was removed from the block surface after each image. The final image series was aligned within the TrakEM2 plugin (Fiji) and then segmented using the arealist function. The final models were then imported into the Blender software (www.blender.org) and the volumes of mitochondria measured using the NeuroMorph toolset (Jorstad et al., 2015) (www.neuromorph.epfl.ch).
Bioinformatic and statistical analysis: For the *in silico* correlation of Pum2 expression levels with longevity, we used publicly available longevity data from the BXD (mean longevity) and LXS genetic reference population (maximal longevity) downloaded from GeneNetwork.org. Pearson's *r* was used to establish correlations between longevity and *Pum2* mRNA levels in whole brain and neocortex respectively. For the GSEA, RNAseq data from human tissues, as well as the covariates data, were downloaded from the GTEx Portal (version v6p) (GTEx Consortium, 2013). Gene expression residuals after removing the available covariates by PEER (Stegle et al., 2012) were used for the enrichment analysis. For enrichment analysis, genes were ranked based on their Pearson correlation coefficients with the expression residuals of *PUM2,* and Gene Set Enrichment Analysis (GSEA) was performed to find the enriched gene sets co-expressed with *PUM2* (Subramanian et al., 2005) by using R/fgsea package (Sergushichev, 2016). Heatmaps were obtained using the heatmap2 function of R/gplots. The network analysis of the proteins induced upon *PUM2* knock-down form the SILAC experiment was performed using Cytoscape 7.0 with the STRING interaction database. Pathway enrichment analysis for Gene Ontology (GO) Cellular Component and for Reactome was performed using EnrichR (http://amp.pharm.mssm.edu/Enrichr/).

Quantification and Statistical Analysis: No statistical methods were used to predetermine sample size. Differences between two groups were assessed using two-tailed t-tests, unless stated otherwise. Differences between more than two groups were assessed by using one-way ANOVA. For worm lifespan analysis, log-rank (Mantel-Cox) test was used to compare two different groups. GraphPad Prism 6 (GraphPad Software, Inc.) was used for all statistical analyses. Variability in plots and graphs is presented as the standard deviation (s.d.), unless stated otherwise. All p<•0.05 were considered to be significant. *p•<•0.05; **p•••0.01; ***p•••0.001; ****p•••0.0001 unless stated otherwise. Mouse experiments were performed once. Animals that showed signs of severity, predefined by the animal authorizations, were euthanized. These animals, together with those who died spontaneously during the experiments, were excluded from the calculations. These criteria were established before starting the experiments. For motility, fitness and death scoring experiments in C. elegans, sample size was estimated based on the known variability of the assay. All experiments were done non-blinded and repeated at least twice.

### Results

### Pum2 is a negative regulator of lifespan.

The inventors analysed the expression of the repertoire of known mammalian RBPs (Neelamraju et al., 2015) in extant transcriptome data from aged mouse muscle tissues (Edwards et al., 2007) (Figure S1A) and found that only the expression of 66 out of 1343 RBPs was significantly changed in both young *vs.* old and old *vs.* old calorie restricted (CR) muscle samples (Figure S1B). *Pumilio2* (*Pum2*) showed a robust increase upon aging, which was restored to the level of young mice in aged CR muscles (Figures S1B and C). *Pum2, Rbm6* and *Nudt16l* are furthermore the only three common regulated RBP transcripts between three datasets (Figure 1A), i.e. aged mouse muscle (Edwards et al., 2007) (Figure SIC), neocortex of aged mice (Oberdoerffer et al., 2008) (Figure S1C and D), and muscle biopsies of aged humans (Yang et al., 2015).Among these three candidates, *PUM2* is the only transcript whose levels are consistently up-regulated upon aging in a skeletal muscle compendium of gene expression in human biopsies (Su et al., 2015). We confirmed the increase of PUM2 expression also at the protein level in quadriceps (Figure 1B), forelimb (Figure 1C) and tibialis (Figure S1F) muscles of aged C57BL/6J mice. PUM2 levels were also increased in the neocortex of old mice (Figure 1D). Of importance, *Pum2* levels in neocortex and whole brain were inversely correlated to mean longevity, in the BXD (Gelman et al., 1988) and in the LXS (Liao et al., 2010) mouse genetic reference populations (Figure IE), suggesting a negative effect of the increase of PUM2 on the aging process.

To experimentally validate this suggestive effect on lifespan, we used the short-lived nematode *C. elegans.* Phylogenetic analysis indicated that *puf-8* was the closest orthologue of mammalian *Pum2* (Spassov and Jurecic, 2003). In wild-type Bristol N2 worms, *puf-8* transcript levels gradually increased upon aging (Figure 1F), consistent with the observations in mice and humans. *puf-8* loss-of-function (LOF) (Figure S1G) leads to a robust lifespan reduction (Figure S1H), due to the known effects of its depletion on germline maturation and on development (Subramaniam and Seydoux, 2003). To avoid these pleiotropic effects of *puf-8* LOF we used germline deficient *glp-1(e2144)* mutant worms to test its impact on lifespan. As in N2 worms, *puf-8* levels are increased in an age-dependent manner in *glp-1* mutants (Figure S1I). Moreover, in the *glp-1* mutants, *puf-8* LOF increased lifespan in contrast to its effect in N2 controls (Figure 1G). This effect was accompanied by a significant increase in fitness (Figure S1J) and mitochondrial respiration in the old worms (Figure 1H), indicating an enhanced healthspan upon *puf-8* RNAi. Thus, these results suggest that the accumulation of both *Pum2* and of its *C*. *elegans* orthologue, *puf-8,* could contribute to accelerate the aging process.

### Multi-omics analyses reveal MFF as a novel post-transcriptional target of PUM2

PUM2 mainly acts as a repressor of protein synthesis, by binding and inhibiting the translation of mRNAs containing the 5•-UGUAXAUA-3• Pumilio-binding element (PBE) (Hafner et al., 2010) and it was previously linked to germ-cell development (Fox et al., 2005), genome stability (Lee et al., 2016), stem cell function (Shigunov et al., 2012) and synaptic morphogenesis (Vessey et al., 2010). Moreover, PUM2 harbours LC regions that upon stress, allow it to form stress granules, in which its target mRNAs are trapped and unable to be translated (Vessey et al., 2010). To further understand PUM2's biological role, we performed a multi-tissue GSEA (gene set enrichment analysis) (Subramanian et al., 2005) in the human GTEx cohort (GTEx Consortium, 2013). *PUM2* transcript levels positively correlate with genes in the gene ontology (GO) categories to which PUM2 belongs, such as "Cytoplasmic stress granule" and "Regulation of post-transcriptional gene silencing" (Figure 2A and Figure S2A and B). Strikingly, all the top 15 GO categories negatively correlating with *PUM2* are linked to mitochondrial function (Figure S2A), except the category "Ribosomal subunits", which could be, however, in line with PUM2's role as a translation repressor. The correlation between *PUM2* and mitochondrial-related categories was confirmed also with tissue-specific GSEA in brain cortex (Figure 2B) and frontal cortex (Figure S2C).

Previous studies on PUM2 function in mammals were mainly based on either transcriptomic or ribonomic analyses (Bohn et al., 2017; Hafner et al., 2010; Lee et al., 2016; Shigunov et al., 2012), inciting us to apply a multi-omics approach to reveal novel post-transcriptional effects of PUM2 that could link it to mitochondrial functions. First, we performed quantitative proteomics after stable isotope labelling with amino acids (SILAC) in HeLa cells. We identified 4485 proteins, of which 85 are commonly up-regulated and 58 down-regulated, in *PUM2* knock-down (*PUM2*sh) versus control (SCR) cells (Figure 2C). The higher number of induced *vs*. repressed proteins is consistent with the removal of PUM2 as a translation inhibitor. The network of up-regulated targets showed a tight functional interaction (Figure S2D) and was significantly enriched for metabolic pathways (Figure 2D). In line with the results of GSEA, the cellular component enrichment analysis suggests that these changes could be driven by mitochondrial-dependent mechanisms (Figure 2E). Consistent with the proteomic analysis, also untargeted metabolomics of *PUM2*sh *vs.* control cells showed that mitochondrial and metabolic pathways, such as TCA cycle and purine/pyrimidine pathways were among the top hits (Figures 2F and S2E and F).

We also performed RNA-Seq to discriminate between transcriptional and post-transcriptional responses driven by *PUM2* depletion. Multi-layered analysis showed only 36 candidates upregulated at protein, but not at mRNA level (Figure 2G). Finally, to identify the direct PUM2 targets leading to these changes, we crossed the proteins from the multi-omics analysis with the list of client transcripts bound by PUM2 in extant human CLIP-Seq data (Hafner et al., 2010). Nine candidates are both bound and repressed by PUM2 (Figure 2G - red color) and only two of them have a perfect PBE in their 3'UTR (Figure 2H). These two hits are potential translational targets of PUM2 and include mitochondrial fission factor (MFF), an outer mitochondrial membrane (OMM) protein required for mitochondrial fission by recruiting the dynamin-like protein DNM1L (Otera et al., 2010) and SLC6A6, a plasma membrane taurine-alanine transporter, whose up-regulation is in line with the increase of taurine and alanine in *PUM2*sh cells (Figure S2G). MFF contains the most highly conserved PBE in different species, including in the *C*. *elegans* MFF orthologue, *mff-1* (Figure 2I). This evidence, together with its direct role in mitochondrial function, pinpoints MFF as a potential mediator of PUM2's effects on mitochondrial homeostasis.

### PUM2 regulates mitochondrial dynamics and mitophagy via MFF

We validated whether PUM2 negatively regulates the MFF protein levels, using two independent shRNAs in mouse C2C12 myoblasts (Figure 3A) and human HeLa cells (Figure S3A). *Pum2* depletion increased MFF at the protein, but not transcript levels in both C2C12 (Figure 3A and 3B) and HeLa cells (Figure S3A and S3B), supporting its role in post-transcriptional regulation. *Mff* has a stable 3'UTR, a feature of post-translationally regulated mRNAs (Figure S3C). *Pum2* depletion in C2C12 cells increased the luciferase activity of a reporter construct under the control of *Mff* 3•UTR sequences (Figure 3C), and cross-linking and immunoprecipitation (CLIP)-qPCR of PUM2 demonstrated its binding on *Mff* mRNA, but not *Actb,* 3'UTR (Figure 3D and Figure S3D). Altogether, these data demonstrate that PUM2 binds and specifically represses the translation of *Mff* mRNA.

Increased MFF levels are reported to cause a shift of mitochondrial dynamics towards fission (Otera et al., 2010). In line with this premise, PUM2 depletion increased the percentage of myoblast cells undergoing fission (Figure 3E and Figure S3E). This effect is MFF-dependent, since the double knock-down of both *Pum2* and *Mff* restores mitochondrial morphology to the basal state (Figures 3E and S3E). Increased fission is accompanied by improved removal of fragmented mitochondria through mitophagy (Frank et al., 2012; MacVicar and Lane, 2014; Tanaka et al., 2010; Twig et al., 2008). Indeed, C2C12 cells treated with two different *Pum2* shRNAs show a reduced mitochondrial to nuclear DNA ratio (mtDNA/nDNA) (Figure 3F), suggesting a higher mitochondrial turnover rate. In addition, mitophagy rates in these clones were assessed by using the mitophagy marker m-KEIMA (Katayama et al., 2011). When mitochondria are at neutral pH m-KEIMA is excited at 440 nm (pseudocolored in cyan), whereas in mitochondria undergoing mitophagy the fluorophore is ionized and changes its excitation wavelength (represented in red). Consistent with the previous data, C2C12 *Pum2sh* clones exhibit increased mitophagy rates, i.e. red to cyan signal, compared to control cells (Figures 3G and Figure S3F). Our data hence unveiled that PUM2 regulates mitochondrial dynamics and suggest that the increase of PUM2 that we observed upon aging could impair mitochondrial dynamics and mitophagy, both hallmarks of aging (Sebastián et al., 2017; Sun et al., 2016).

### Depletion of puf-8 improves mitochondrial dynamics and function during nematode aging

Aging is accompanied by the accumulation of giant, dysfunctional mitochondria, that cannot be eliminated by mitophagy, as reported in muscles of nematodes (Yasuda et al., 2006), flies (Rana et al., 2017) and mice (Leduc-Gaudet et al., 2015; Mercken et al., 2017). We confirmed the increase of swollen mitochondria in old (Day 8) relative to young (Day 1) worms that express p_{myo-3}::mito::GFP, a GFP localized in the mitochondria of body wall muscles (Figures 4A and B). Importantly, *puf-8* RNAi feeding from the adult stage reverted this aging phenotype (Day 8, *puf-8*) and re-established mitochondrial networks similar to those of young worms. In addition, double RNAi of both *puf-8* and *mff-1* cancelled the normalizing effect of *puf-8* LOF on mitochondrial morphology (Figures 4A). *puf-8* depletion also increased the expression of the mitophagy markers, *dct-1, pink-1* and *pdr-1* (Figure 4B) and of several OXPHOS genes (Figure 4C), in an *mff-1*-dependent fashion. Interestingly, no changes were observed in markers of mitochondrial and endoplasmic reticulum unfolded protein response (UPR^{mt} and UPR^{er}, respectively), oxidative stress and heat shock response (HSR) (Figure S4A), supporting a predominant role of *puf-8* in mitochondrial dynamics and mitophagy. Importantly, the effects of the *puf-8*/*mff-1* axis on mitochondria also impact on the general muscle homeostasis, since *puf-8* worms showed reduced aging-related muscle fiber damage in an *mff-1* dependent fashion (Figures 4D and S4B). These data demonstrate an evolutionary conserved role of PUM2 in regulating mitochondrial fission and mitophagy through MFF and show that *puf-8* contributes to aging-dependent alterations in mitochondrial dynamics in *C*. *elegans.*

### PUM2 knock-down in old mice improves mitochondrial function

To ascertain whether PUM2 inhibition affects mitochondrial dynamics and quality also during mammalian aging in a fashion similar to nematodes, we knocked-down *PUM2* in muscles of old mice *in vivo* using the CRISPR-Cas9 system (Ran et al., 2015). First, we tested three different *Pum2*-targeting guides (*gPum2*) in 293T cells and selected the guide leading to the most efficient knock-down of PUM2 and consequent upregulation of its target, MFF (g*PUM2* #1, Figure S5A). Genomic sequencing of the region around the gRNA-targeted site confirmed the efficient genome editing of *PUM2* gene (Figures S5B and C). We then injected Cas9/*gPum2* or Cas9 (as a control) encoding AAV9s, bilaterally, in gastrocnemius muscles of 24 month old C57BL/6J mice. *Pum2* depletion in the muscle of old mice increased the MFF protein (Figure 5A), but not the mRNA level (Figure S5D) and induced the fragmentation (Figure 5B) and reduction in volume fraction (Figure 5C and D and Movies S1 and S2) of hyperfused and swollen muscle intermyofibrillar (IFM) mitochondria that are typical for aged muscle fibers. Similarly to what we observed in nematodes, *Pum2* knock-down in the gastrocnemius increased the levels of a subset of OXPHOS proteins (Figure 5E) and of citrate synthase activity (Figure 5F), reflecting a better mitochondrial function. Consistent with the data in cells and worms, LOF of PUM2 also affects mitophagy *in vivo.* Indeed, depletion of *Pum2* stabilized PINK1 protein levels, increased the LC3-II/LC3-I ratio, and decreased p62/SQSTM levels (Figures 5G and H), three markers of increased autophagy/mitophagy (Okatsu et al., 2010; Ryu et al., 2016; Burman et al., 2017; Sorrentino et al., 2017). Altogether, the CRISPR-Cas9 genome editing of *Pum2* in the muscle of elderly mice shows that PUM2 regulates mitochondrial dynamics and function, as well as mitochondrial clearance upon aging in a similar fashion as in *C*. *elegans.*

### PUM2 sequesters Mff mRNA in aging-associated stress granules

Upon aging, certain RBPs are reported to form aggregates, with detrimental impact on lifespan in both yeast (Schlissel et al., 2017) and *C*. *elegans* (Lechler et al., 2017). Little is known whether these "aging-granules" have a conserved function in mammals. We hence tested whether PUM2 could sequester and inhibit the translation of specific mRNAs, such as *Mff,* in stress granules (SGs) upon aging. We co-stained C2C12 myoblasts for both PUM2 protein and *Mff* mRNA, in basal conditions and upon treatment with the SG-inducer sodium arsenite (NaAsO₂). In non-stressed conditions, PUM2 was uniformly distributed and *Mff* mRNA was randomly scattered throughout the cytosol. However, after exposure to NaAsO₂, PUM2 formed discrete stress granules and *Mff* mRNA relocalized in the proximity to these PUM2-SGs (Figure 6A). This association did not occur when using a probe targeting the control mRNA, *Ppib,* coding for the Cyclophillin B protein, which lacked a PUM2 binding site (Figure S6A). A probe targeting the bacterial mRNA, *Dabp,* as negative control, showed no signal, validating the specificity of the mRNA staining (Figure S6B).
When certain SG-components are highly concentrated they spontaneously tend to form larger and more stable aggregates (Molliex et al., 2015). Indeed, a GFP-tagged PUM2 protein expressed at low concentrations mostly exhibited a uniform staining in the cytosol (Figure 6B, top), similarly to the endogenous PUM2 (Figure 6A, top). Increasing the expression of GFP-PUM2, however, dose-dependently resulted in more and bigger condensates that colocalized with the endogenous SG-component PABPC1 (red) (Figures 6B, C and D). This effect was not due to the high expression of the GFP-tag, since expression of the GFP alone at the same higher dose, did not result in aggregate formation or association with PABPC1 (Figure S6C). In combination, these data suggest that high levels of PUM2 favour the formation of large and persistent SGs, where mRNA targets are trapped and inhibited.

The immunoprecipitation of endogenous PUM2 from the heavy RNPs-containing fractions (fractions 4 to 7 from Figure 7A) in aged brain tissues showed that PUM2 interacts with the SGs protein PABPC1 (Figure 7B) and the *Mff* mRNA (Figure 7C). Consistently, PUM2 colocalized with Thioflavin S (ThS), a dye which stains amyloidogenic SGs (Molliex et al., 2015) in the neocortex (Figure 7D), cerebellum (Figure S6E) and dentate gyrus (Figure S6F). Furthermore, the number (Figure 7E) and size (Figure 7F) of the PUM2 SGs was significantly increased when comparing young to old animals. Altogether, these data show that upon aging PUM2 accumulates in RNPs together with other SGs components. In these aging SGs, PUM2 binds and sequesters *Mff,* repressing its translation, thereby impairing mitochondrial fission, mitophagy and ultimately leading to age-associated mitochondrial dysfunction (Figure 7G).

### REFERENCES

Abeliovich, H., Zarei, M., Rigbolt, K.T.G., Youle, R.J., and Dengjel, J. (2013). Involvement of mitochondrial dynamics in the segregation of mitochondrial matrix proteins during stationary phase mitophagy. Nat. Commun. 4, 2789.
Apicco, D.J., Ash, P.E.A., Maziuk, B., LeBlang, C., Medalla, M., Abdullatif, A.A., Ferragud, A., Botelho, E., Ballance, H.I., Dhawan, U., et al. (2018). Reducing the RNA binding protein TIA1 protects against tau-mediated neurodegeneration in vivo. Nat. Neurosci. 21, 72.
Banerjee, A., Apponi, L.H., Pavlath, G.K., and Corbett, A.H. (2013). PABPN1: molecular function and muscle disease. FEBS J. 280, 4230-4250.
Bohn, J.A., Van Etten, J.L., Schagat, T.L., Bowman, B.M., McEachin, R.C., Freddolino, P.L., and Goldstrohm, A.C. (2017). Identification of diverse target RNAs that are functionally regulated by human Pumilio proteins. Nucleic Acids Res.
Burman, J.L., Pickles, S., Wang, C., Sekine, S., Vargas, J.N.S., Zhang, Z., Youle, A.M., Nezich, C.L., Wu, X., Hammer, J.A., et al. (2017). Mitochondrial fission facilitates the selective mitophagy of protein aggregates. J Cell Biol jcb.201612106.
Chaturvedi, P., Neelamraju, Y., Arif, W., Kalsotra, A., and Janga, S.C. (2015). Uncovering RNA binding proteins associated with age and gender during liver maturation. Sci. Rep. 5, 9512.
Conlon, E.G., and Manley, J.L. (2017). RNA-binding proteins in neurodegeneration: mechanisms in aggregate. Genes Dev. 31, 1509-1528.
D'Amico, D., Sorrentino, V., and Auwerx, J. (2017). Cytosolic Proteostasis Networks of the Mitochondrial Stress Response. Trends Biochem. Sci. 42, 712-725.
D'Costa, S., Blouin, V., Broucque, F., Penaud-Budloo, M., François, A., Perez, I.C., Le Bec, C., Moullier, P., Snyder, R.O., and Ayuso, E. (2016). Practical utilization of recombinant AAV vector reference standards: focus on vector genomes titration by free ITR qPCR. Mol. Ther. Methods Clin. Dev. 5, 16019.
Diot, A., Morten, K., and Poulton, J. (2016). Mitophagy plays a central role in mitochondrial ageing. Mamm. Genome 27, 381-395.
Edwards, M.G., Anderson, R.M., Yuan, M., Kendziorski, C.M., Weindruch, R., and Prolla, T.A. (2007). Gene expression profiling of aging reveals activation of a p53-mediated transcriptional program. BMC Genomics 8, 80.
Fox, M., Urano, J., and Reijo Pera, R.A. (2005). Identification and characterization of RNA sequences to which human PUMILIO-2 (PUM2) and deleted in Azoospermia-like (DAZL) bind. Genomics 85, 92-105.
Frank, M., Duvezin-Caubet, S., Koob, S., Occhipinti, A., Jagasia, R., Petcherski, A., Ruonala, M.O., Priault, M., Salin, B., and Reichert, A.S. (2012). Mitophagy is triggered by mild oxidative stress in a mitochondrial fission dependent manner. Biochim. Biophys. Acta 1823, 2297-2310.
Fritzsche, R., Karra, D., Bennett, K.L., Ang, F. yee, Heraud-Farlow, J.E., Tolino, M., Doyle, M., Bauer, K.E., Thomas, S., Planyavsky, M., et al. (2013). Interactome of Two Diverse RNA Granules Links mRNA Localization to Translational Repression in Neurons. Cell Rep. 5, 1749-1762.
Gelman, R., Watson, A., Bronson, R., and Yunis, E. (1988). Murine Chromosomal Regions Correlated with Longevity. Genetics 118, 693-704.
Gitler, A.D., and Shorter, J. (2011). RNA-binding proteins with prion-like domains in ALS and FTLD-U. Prion 5, 179-187.
Gonskikh, Y., and Polacek, N. (2017). Alterations of the translation apparatus during aging and stress response. Mech. Ageing Dev. 168, 30-36.
GTEx Consortium (2013). The Genotype-Tissue Expression (GTEx) project. Nat. Genet. 45, 580-585.
Hafner, M., Landthaler, M., Burger, L., Khorshid, M., Hausser, J., Berninger, P., Rothballer, A., Ascano, M., Jungkamp, A.-C., Munschauer, M., et al. (2010). Transcriptome-wide identification of RNA-binding protein and microRNA target sites by PAR-CLIP. Cell 141, 129-141.
Harrison, A.F., and Shorter, J. (2017). RNA-binding proteins with prion-like domains in health and disease. Biochem. J. 474, 1417-1438.
Houtkooper, R.H., Williams, R.W., and Auwerx, J. (2010). Metabolic Networks of Longevity. Cell 142, 9-14.
Houtkooper, R.H., Mouchiroud, L., Ryu, D., Moullan, N., Katsyuba, E., Knott, G., Williams, R.W., and Auwerx, J. (2013). Mitonuclear protein imbalance as a conserved longevity mechanism. Nature 497, 451-457.
Huang, E.J., Zhang, J., Geser, F., Trojanowski, J.Q., Strober, J.B., Dickson, D.W., Brown, R.H., Shapiro, B.E., and Lomen-Hoerth, C. (2010). Extensive FUS-immunoreactive Pathology in Juvenile Amyotrophic Lateral Sclerosis with Basophilic Inclusions. Brain Pathol. Zurich Switz. 20, 1069-1076.
Jorstad, A., Nigro, B., Cali, C., Wawrzyniak, M., Fua, P., and Knott, G. (2015). NeuroMorph: A Toolset for the Morphometric Analysis and Visualization of 3D Models Derived from Electron Microscopy Image Stacks. Neuroinformatics 13, 83-92.
Kageyama, Y., Hoshijima, M., Seo, K., Bedja, D., Sysa-Shah, P., Andrabi, S.A., Chen, W., Höke, A., Dawson, V.L., Dawson, T.M., et al. (2014). Parkin-independent mitophagy requires Drpl and maintains the integrity of mammalian heart and brain. EMBO J. 33, 2798-2813.
Kamath, R.S., Martinez-Campos, M., Zipperlen, P., Fraser, A.G., and Ahringer, J. (2001). Effectiveness of specific RNA-mediated interference through ingested double-stranded RNA in Caenorhabditis elegans. Genome Biol. 2, RESEARCH0002.
Katayama, H., Kogure, T., Mizushima, N., Yoshimori, T., and Miyawaki, A. (2011). A Sensitive and Quantitative Technique for Detecting Autophagic Events Based on Lysosomal Delivery. Chem. Biol. 18, 1042-1052.
Koopman, M., Michels, H., Dancy, B.M., Kamble, R., Mouchiroud, L., Auwerx, J., Nollen, E.A.A., and Houtkooper, R.H. (2016). A screening-based platform for the assessment of cellular respiration in Caenorhabditis elegans. Nat. Protoc. 11, 1798-1816.
Lechler, M.C., Crawford, E.D., Groh, N., Widmaier, K., Jung, R., Kirstein, J., Trinidad, J.C., Burlingame, A.L., and David, D.C. (2017). Reduced Insulin/IGF-1 Signaling Restores the Dynamic Properties of Key Stress Granule Proteins during Aging. Cell Rep. 18, 454-467.
Leduc-Gaudet, J.-P., Picard, M., Pelletier, F.S.-J., Sgarioto, N., Auger, M.-J., Vallée, J., Robitaille, R., St-Pierre, D.H., Gouspillou, G., Leduc-Gaudet, J.-P., et al. (2015). Mitochondrial morphology is altered in atrophied skeletal muscle of aged mice. Oncotarget 6, 17923-17937.
Lee, S., Kopp, F., Chang, T.-C., Sataluri, A., Chen, B., Sivakumar, S., Yu, H., Xie, Y., and Mendell, J.T. (2016). Noncoding RNA NORAD Regulates Genomic Stability by Sequestering PUMILIO Proteins. Cell 164, 69-80.
Lee, Y., Jonson, P.H., Sarparanta, J., Palmio, J., Sarkar, M., Vihola, A., Evilä, A., Suominen, T., Penttilä, S., Savarese, M., et al. (2018). TIA1 variant drives myodegeneration in multisystem proteinopathy with SQSTM1 mutations. J. Clin. Invest. 128*.*
Liao, C.-Y., Rikke, B.A., Johnson, T.E., Diaz, V., and Nelson, J.F. (2010). Genetic Variation in the Murine Lifespan Response to Dietary Restriction: from Life Extension to Life Shortening. Aging Cell 9, 92-95.
Löw, K., Aebischer, P., and Schneider, B.L. (2013). Direct and retrograde transduction of nigral neurons with AAV6, 8, and 9 and intraneuronal persistence of viral particles. Hum. Gene Ther. 24, 613-629.
MacVicar, T.D.B., and Lane, J.D. (2014). Impaired OMA1-dependent cleavage of OPA1 and reduced DRP1 fission activity combine to prevent mitophagy in cells that are dependent on oxidative phosphorylation. J Cell Sci 127, 2313-2325.
Masuda, K., Marasa, B., Martindale, J.L., Halushka, M.K., and Gorospe, M. (2009). Tissue- and age-dependent expression of RNA-binding proteins that influence mRNA turnover and translation. Aging 1, 681-698.
Mercken, E.M., Capri, M., Carboneau, B.A., Conte, M., Heidler, J., Santoro, A., Martin-Montalvo, A., Gonzalez-Freire, M., Khraiwesh, H., González-Reyes, J.A., et al. (2017). Conserved and species-specific molecular denominators in mammalian skeletal muscle aging. Npj Aging Mech. Dis. 3, 8.
Molliex, A., Temirov, J., Lee, J., Coughlin, M., Kanagaraj, A.P., Kim, H.J., Mittag, T., and Taylor, J.P. (2015). Phase separation by low complexity domains promotes stress granule assembly and drives pathological fibrillization. Cell 163, 123-133.
Mouchiroud, L., Houtkooper, R.H., Moullan, N., Katsyuba, E., Ryu, D., Cantó, C., Mottis, A., Jo, Y.-S., Viswanathan, M., Schoonjans, K., et al. (2013). The NAD+/Sirtuin Pathway Modulates Longevity through Activation of Mitochondrial UPR and FOXO Signaling. Cell 154, 430-441.
Mouchiroud, L., Sorrentino, V., Williams, E.G., Cornaglia, M., Frochaux, M.V., Lin, T., Nicolet-Dit-Félix, A.A., Krishnamani, G., Ouhmad, T., Gijs, M.A.M., et al. (2016). The Movement Tracker: A Flexible System for Automated Movement Analysis in Invertebrate Model Organisms. Curr. Protoc. Neurosci. 77, 8.37.1-8.37.21.
Neelamraju, Y., Hashemikhabir, S., and Janga, S.C. (2015). The human RBPome: from genes and proteins to human disease. J. Proteomics 127, 61-70.
Oberdoerffer, P., Michan, S., McVay, M., Mostoslavsky, R., Vann, J., Park, S.-K., Hartlerode, A., Stegmuller, J., Hafner, A., Loerch, P., et al. (2008). SIRT1 redistribution on chromatin promotes genomic stability but alters gene expression during aging. Cell 135, 907-918.
Okatsu, K., Saisho, K., Shimanuki, M., Nakada, K., Shitara, H., Sou, Y., Kimura, M., Sato, S., Hattori, N., Komatsu, M., et al. (2010). P62/SQSTM1 cooperates with Parkin for perinuclear clustering of depolarized mitochondria. Genes Cells 15, 887-900.
Otera, H., Wang, C., Cleland, M.M., Setoguchi, K., Yokota, S., Youle, R.J., and Mihara, K. (2010). Mff is an essential factor for mitochondrial recruitment of Drp1 during mitochondrial fission in mammalian cells. J. Cell Biol. 191, 1141-1158.
Ran, F.A., Cong, L., Yan, W.X., Scott, D.A., Gootenberg, J.S., Kriz, A.J., Zetsche, B., Shalem, O., Wu, X., Makarova, K.S., et al. (2015). In vivo genome editing using Staphylococcus aureus Cas9. Nature 520, 186-191.
Rana, A., Rera, M., and Walker, D.W. (2013). Parkin overexpression during aging reduces proteotoxicity, alters mitochondrial dynamics, and extends lifespan. Proc. Natl. Acad. Sci. U. S. A. 110, 8638-8643.
Rana, A., Oliveira, M.P., Khamoui, A.V., Aparicio, R., Rera, M., Rossiter, H.B., and Walker, D.W. (2017). Promoting Drpl-mediated mitochondrial fission in midlife prolongs healthy lifespan of Drosophila melanogaster. Nat. Commun. 8, 448.
Ryu, D., Mouchiroud, L., Andreux, P.A., Katsyuba, E., Moullan, N., Nicolet-dit-Felix, A.A., Williams, E.G., Jha, P., Lo Sasso, G., Huzard, D., et al. (2016). Urolithin A induces mitophagy and prolongs lifespan in C. elegans and increases muscle function in rodents. Nat. Med. 22, 879-888.
Schlissel, G., Krzyzanowski, M.K., Caudron, F., Barral, Y., and Rine, J. (2017). Aggregation of the Whi3 protein, not loss of heterochromatin, causes sterility in old yeast cells. Science 355, 1184-1187.
Sebastián, D., Palacín, M., and Zorzano, A. (2017). Mitochondrial Dynamics: Coupling Mitochondrial Fitness with Healthy Aging. Trends Mol. Med. 23, 201-215.
Sergushichev, A. (2016). An algorithm for fast preranked gene set enrichment analysis using cumulative statistic calculation. BioRxiv 060012.
Sheinberger, J., and Shav-Tal, Y. (2017). mRNPs meet stress granules. FEBS Lett. 591, 2534-2542.
Shi, Z., and Barna, M. (2015). Translating the genome in time and space: specialized ribosomes, RNA regulons, and RNA-binding proteins. Annu. Rev. Cell Dev. Biol. 31, 31-54.
Shigunov, P., Sotelo-Silveira, J., Kuligovski, C., de Aguiar, A.M., Rebelatto, C.K., Moutinho, J.A., Brofman, P.S., Krieger, M.A., Goldenberg, S., Munroe, D., et al. (2012). PUMILIO-2 is involved in the positive regulation of cellular proliferation in human adipose-derived stem cells. Stem Cells Dev. 21, 217-227.
Sonntag, F., Köther, K., Schmidt, K., Weghofer, M., Raupp, C., Nieto, K., Kuck, A., Gerlach, B., Böttcher, B., Müller, O.J., et al. (2011). The assembly-activating protein promotes capsid assembly of different adeno-associated virus serotypes. J. Virol. 85, 12686-12697.
Sorrentino, V., Romani, M., Mouchiroud, L., Beck, J.S., Zhang, H., D'Amico, D., Moullan, N., Potenza, F., Schmid, A.W., Rietsch, S., et al. (2017). Enhancing mitochondrial proteostasis reduces amyloid-• proteotoxicity. Nature 552, 187-193.
Souquere, S., Mollet, S., Kress, M., Dautry, F., Pierron, G., and Weil, D. (2009). Unravelling the ultrastructure of stress granules and associated P-bodies in human cells. J. Cell Sci. 122, 3619-3626.
Spassov, D., and Jurecic, R. (2003). The PUF Family of RNA-binding Proteins: Does Evolutionarily Conserved Structure Equal Conserved Function? IUBMB Life 55, 359-366.
Steffen, K.K., and Dillin, A. (2016). A Ribosomal Perspective on Proteostasis and Aging. Cell Metab. 23, 1004-1012.
Stegle, O., Parts, L., Piipari, M., Winn, J., and Durbin, R. (2012). Using probabilistic estimation of expression residuals (PEER) to obtain increased power and interpretability of gene expression analyses. Nat. Protoc. 7, 500-507.
Su, J., Ekman, C., Oskolkov, N., Lahti, L., Ström, K., Brazma, A., Groop, L., Rung, J., and Hansson, O. (2015). A novel atlas of gene expression in human skeletal muscle reveals molecular changes associated with aging. Skelet. Muscle 5, 35.
Subramaniam, K., and Seydoux, G. (2003). Dedifferentiation of primary spermatocytes into germ cell tumors in C. elegans lacking the pumilio-like protein PUF-8. Curr. Biol. CB 13, 134-139.
Subramanian, A., Tamayo, P., Mootha, V.K., Mukherjee, S., Ebert, B.L., Gillette, M.A., Paulovich, A., Pomeroy, S.L., Golub, T.R., Lander, E.S., et al. (2005). Gene set enrichment analysis: a knowledge-based approach for interpreting genome-wide expression profiles. Proc. Natl. Acad. Sci. U. S. A. 102, 15545-15550.
Sun, N., Yun, J., Liu, J., Malide, D., Liu, C., Rovira, I.I., Holmström, K.M., Fergusson, M.M., Yoo, Y.H., Combs, C.A., et al. (2015). Measuring In Vivo Mitophagy. Mol. Cell 60, 685-696.
Sun, N., Youle, R.J., and Finkel, T. (2016). The Mitochondrial Basis of Aging. Mol. Cell 61, 654-666.
Tanaka, A., Cleland, M.M., Xu, S., Narendra, D.P., Suen, D.-F., Karbowski, M., and Youle, R.J. (2010). Proteasome and p97 mediate mitophagy and degradation of mitofusins induced by Parkin. J. Cell Biol. 191, 1367-1380.
Twig, G., Elorza, A., Molina, A.J.A., Mohamed, H., Wikstrom, J.D., Walzer, G., Stiles, L., Haigh, S.E., Katz, S., Las, G., et al. (2008). Fission and selective fusion govern mitochondrial segregation and elimination by autophagy. EMBO J. 27, 433-446.
Tyanova, S., Temu, T., Sinitcyn, P., Carlson, A., Hein, M.Y., Geiger, T., Mann, M., and Cox, J. (2016). The Perseus computational platform for comprehensive analysis of (prote)omics data. Nat. Methods 13, 731-740.
Vessey, J.P., Schoderboeck, L., Gingl, E., Luzi, E., Riefler, J., Di Leva, F., Karra, D., Thomas, S., Kiebler, M.A., and Macchi, P. (2010). Mammalian Pumilio 2 regulates dendrite morphogenesis and synaptic function. Proc. Natl. Acad. Sci. U. S. A. 107, 3222-3227.
Wishart, D.S., Jewison, T., Guo, A.C., Wilson, M., Knox, C., Liu, Y., Djoumbou, Y., Mandal, R., Aziat, F., Dong, E., et al. (2013). HMDB 3.0-The Human Metabolome Database in 2013. Nucleic Acids Res. 41, D801-D807.
Winiewski, J.R., Zougman, A., Nagaraj, N., and Mann, M. (2009). Universal sample preparation method for proteome analysis. Nat. Methods 6, 359.
Yang, J., Huang, T., Petralia, F., Long, Q., Zhang, B., Argmann, C., Zhao, Y., Mobbs, C.V., Schadt, E.E., Zhu, J., et al. (2015). Synchronized age-related gene expression changes across multiple tissues in human and the link to complex diseases. Sci. Rep. 5, 15145.
Yasuda, K., Ishii, T., Suda, H., Akatsuka, A., Hartman, P.S., Goto, S., Miyazawa, M., and Ishii, N. (2006). Age-related changes of mitochondrial structure and function in Caenorhabditis elegans. Mech. Ageing Dev. 127, 763-770.
Zheng, L., Bernard-Marissal, N., Moullan, N., D'Amico, D., Auwerx, J., Moore, D.J., Knott, G., Aebischer, P., and Schneider, B.L. (2017). Parkin functionally interacts with PGC-1• to preserve mitochondria and protect dopaminergic neurons. Hum. Mol. Genet. 26, 582-598.

## Claims

1. A pharmaceutical composition comprising at least one agent that reduces the levels and/or activity of the RNA-binding protein PUM2 for use in treating or preventing an aging-related disease.

2. The pharmaceutical composition for use of claim 1, wherein the at least one agent inhibits translation of an RNA encoding PUM2.

3. The pharmaceutical composition for use of claim 1, wherein the at least one agent inhibits transcription of a DNA encoding PUM2.

4. The pharmaceutical composition for use of claim 1 or 2, wherein the at least one agent is a nucleic acid.

5. The pharmaceutical composition for use of claim 4, wherein the nucleic acid is selected from the group comprising a miRNA, a siRNA, a piRNA, a hnRNA, a snRNA, a esiRNA, a shRNA, and an antisense oligonucleotide, or a combination thereof.

6. The pharmaceutical composition for use of claim 1, wherein the at least one agent is a gene delivery vector comprising
i) a Cas9 endonuclease, and
ii) at least one single guide RNA (sgRNA), or crRNA and tracrRNA, recognizing a DNA target sequence comprising 16 to 25 nucleotides wherein said DNA target sequence is selected from the group comprising the *Pum2* gene and a regulatory sequence of the *Pum2* gene.

7. The pharmaceutical composition for use of claim 1, wherein the at least one agent is a gene delivery vector comprising i) a nucleic acid encoding at least one TALEN protein, wherein the at least one TALEN protein is capable of inducing a site-specific double stranded DNA break in a DNA target sequence selected from the group comprising the *Pum2* gene and a regulatory sequence of the *Pum2* gene, and
ii) a nucleic acid donor sequence, wherein the donor sequence is a template for targeting sequence selected from the group comprising the *PUM2* gene and a regulatory sequence of the *PUM2* gene.

8. The pharmaceutical composition for use of claim 6 or 7, wherein the gene delivery vector is selected from the group comprising a viral or a plasmid vector.

9. The pharmaceutical composition for use of claim 4, wherein the nucleic acid is a nucleic acid encoding at least one Cas9 endonuclease, and at least one single guide RNA (sgRNA), or crRNA and tracrRNA, recognizing a DNA target sequence comprising 16 to 25 nucleotides wherein said DNA target sequence is selected from the group comprising the *Pum2* gene and a regulatory sequence of the *Pum2* gene.

10. The pharmaceutical composition for use of claim 4, wherein the nucleic acid is a nucleic acid encoding at least one TALEN protein, wherein the at least one TALEN protein is capable of inducing a site-specific double stranded DNA break in a DNA target sequence selected from the group comprising the *Pum2* gene and a regulatory sequence of the *Pum2* gene; and at least one nucleic acid donor sequence, wherein the donor sequence is a template for targeting sequence selected from the group comprising the *PUM2* gene and a regulatory sequence of the *PUM2* gene

11. The pharmaceutical composition for use of any one of claims 1 to 3, wherein the at least one agent is selected from the group comprising a chemical agent, a peptide or analog thereof, an antibody or an antigen-binding fragment of said antibody.

12. The pharmaceutical composition for use of claim 11, wherein the chemical agent is selected from the group comprising a tetracycline, actinonin, a phenicol, Urolithin A (UA) and Carbonyl cyanide-4-(trifluoromethoxy)phenylhydrazone (FCCP).

13. The pharmaceutical composition for use of any one of the preceding claims, wherein the aging-related disease is selected from the group comprising muscle diseases, such as inclusion body myositis, age-related sarcopenia, frailty of the elderly, and muscular dystrophy's (such as Duchenne's or Becker's muscular dystrophy).

14. The pharmaceutical composition for use of any one of claims 1 to 14, wherein the aging-related disease is a neurodegenerative disease selected from the group comprising Alzheimer's disease, Dementia, Parkinson's disease, Huntington's disease, and amyotrophic lateral sclerosis.

15. A gene delivery vector comprising
i) a Cas9 endonuclease, and ii) at least one single guide RNA (sgRNA), or crRNA and tracrRNA, recognizing a DNA target sequence comprising 16 to 25 nucleotides wherein said DNA target sequence is selected from the group comprising the *Pum2* gene and a regulatory sequence of the *Pum2* gene,
or
i) a nucleic acid encoding at least one TALEN protein, wherein the at least one TALEN protein is capable of inducing a site-specific double stranded DNA break in a DNA target sequence selected from the group comprising the *Pum2* gene and a regulatory sequence of the *Pum2* gene, and ii) at least one nucleic acid donor sequence, wherein the donor sequence is a template for targeting sequence selected from the group comprising the *PUM2* gene and a regulatory sequence of the *PUM2* gene

16. The gene delivery vector of claim 15, wherein the vector is a viral or a plasmid vector.

17. The gene delivery vector of claim 16, wherein the viral vector is selected from the group comprising an adeno-associated virus (AAV) and a lentivirus.

18. The gene delivery vector of claim 16, wherein the plasmid vector is a pAAV based plasmid.

19. A nucleic acid encoding an miRNA, siRNA, piRNA, hnRNA, snRNA, esiRNA, shRNA, or antisense oligonucleotide that reduces the levels and/or activity of the RNA-binding protein PUM2.

20. A pharmaceutical composition comprising at least one agent that reduces the levels and/or activity of the RNA-binding protein PUM2.

21. A method of treating or preventing an aging-related disease, the method comprising administering to a subject in need thereof a pharmaceutical composition comprising a therapeutic or prophylactic amount of at least one agent that reduces the levels and/or activity of the RNA-binding protein PUM2.

22. The method of claim 21, wherein the at least one agent inhibits translation of an RNA encoding PUM2.

23. The method of claim 21, wherein the at least one agent inhibits transcription of a DNA encoding PUM2.

24. The method of claim 21 or 22, wherein the at least one agent is a nucleic acid.

25. The method of claim 4, wherein the nucleic acid is selected from the group comprising a miRNA, a siRNA, a piRNA, a hnRNA, a snRNA, a esiRNA, a shRNA, and an antisense oligonucleotide, or a combination thereof.

26. The method of claim 21, wherein the at least one agent is a gene delivery vector comprising
i) a Cas9 endonuclease, and
ii) at least one single guide RNA (sgRNA), or crRNA and tracrRNA, recognizing a DNA target sequence comprising 16 to 25 nucleotides wherein said DNA target sequence is selected from the group comprising the *Pum2* gene and a regulatory sequence of the *Pum2* gene.

27. The method of claim 21, wherein the at least one agent is a gene delivery vector comprising i) a nucleic acid encoding at least one TALEN protein, wherein the at least one TALEN protein is capable of inducing a site-specific double stranded DNA break in a DNA target sequence selected from the group comprising the *Pum2* gene and a regulatory sequence of the *Pum2* gene, and
ii) a nucleic acid donor sequence, wherein the donor sequence is a template for targeting sequence selected from the group comprising the *PUM2* gene and a regulatory sequence of the *PUM2* gene.

28. The method of claim 26 or 27, wherein the gene delivery vector is selected from the group comprising a viral or a plasmid vector.

29. The method of claim 24, wherein the nucleic acid is a nucleic acid encoding at least one Cas9 endonuclease, and at least one single guide RNA (sgRNA), or crRNA and tracrRNA, recognizing a DNA target sequence comprising 16 to 25 nucleotides wherein said DNA target sequence is selected from the group comprising the *Pum2* gene and a regulatory sequence of the *Pum2* gene.

30. The method of claim 24, wherein the nucleic acid is a nucleic acid encoding at least one TALEN protein, wherein the at least one TALEN protein is capable of inducing a site-specific double stranded DNA break in a DNA target sequence selected from the group comprising the *Pum2* gene and a regulatory sequence of the *Pum2* gene; and at least one nucleic acid donor sequence, wherein the donor sequence is a template for targeting sequence selected from the group comprising the *PUM2* gene and a regulatory sequence of the *PUM2* gene

31. The method of any one of claims 21 to 23, wherein the at least one agent is selected from the group comprising a chemical agent, a peptide or analog thereof, an antibody or an antigen-binding fragment of said antibody.

32. The method of claim 31, wherein the chemical agent is selected from the group comprising a tetracycline, actinonin, a phenicol, Urolithin A (UA) and Carbonyl cyanide-4-(trifluoromethoxy)phenylhydrazone (FCCP).

33. The method of claim 32, wherein the tetracycline is selected from the group comprising doxycycline, tetracycline, rolitetracycline, methacycline, minocycline and lymecycline.

34. The method of claim 32, wherein the phenicol is selected from the group comprising chloramphenicol, thiamphenicol, and florfenicol.

35. The method of any one of the preceding claims, wherein the aging-related disease is selected from the group comprising muscle diseases, such as inclusion body myositis, age-related sarcopenia, frailty of the elderly, and muscular dystrophy's (such as Duchenne's or Becker's muscular dystrophy).

36. The method of any one of claims 21 to 34, wherein the aging-related disease is a neurodegenerative disease.

37. The method of claim 36, wherein the neurodegenerative disease is selected from the group comprising Alzheimer's disease, Dementia, Parkinson's disease, Huntington's disease, and amyotrophic lateral sclerosis.

38. A kit comprising i) a pharmaceutical composition of any one of claims 18 - 14 or 20, ii) a gene delivery vector of anyone of claim 15-18 or iii) the acid nucleic of claim 19 .
